# EUROPEAN PATENT SPECIFICATION

(11) **EP 1 791 820 B1**
(45) Date of publication and mention of the grant of the patent: **02.12.2009**
(21) Application number: 05770221.9
(22) Date of filing: 21.06.2005
(51) Int. Cl.: C07D 215/26, C07D 251/26

(54) **ENANTIOSELECTIVE PREPARATION OF QUINOLINE DERIVATIVES**
ENANTIOSELEKTIVE HERSTELLUNG VON CHINOLIN-DERIVATEN
COMPOSÉS ORGANIQUES

(30) Priority: 22.06.2004 GB 0413960
(43) Date of publication of application: 06.06.2007
(73) Proprietor: Novartis AG, 4056 Basel (CH); Novartis Pharma GmbH, 1230 Wien (AT)
(72) Inventor: LOHSE, Olivier, Ch-4056 Basel (CH); VOGEL, Caspar, CH-4102 Binningen (CH)
(74) Representative: McLean, Craig Sutherland
(86) International application number: PCT/EP2005/006686
(87) International publication number: WO 2005/123684

(56) References cited:
- WO-A-00/75114
- WO-A-2004/076422
- US-A- 5 750 701
- K. PÜNTENER ET. AL.: "New Efficient Catalysts for Enantioselective Transfer Hydrogenations." TETRAHEDRON LETTERS, vol. 37, no. 45, 1996, pages 8165-8168, XP002367502 cited in the application
- KERL-JOSEF HAACK ET. AL.: "The Catalyst Precursor, Catalyst, and Intermediate in the Ru(II) Promoted Asymmetric Hydrogen Transfer Between Alcohols and Ketones." ANGEWANDTE CHEMIE, INTERNATIONAL EDITION IN ENGLISH, vol. 36, no. 3, 1997, pages 285-288, XP002367503 cited in the application

## Description

The present invention provides a practical and high-yielding process for the large scale manufacture of 8-substituted oxy-5-((*R*)-2-halo-1-hydroxy-ethyl)-(*1H*)-quinolin-2-ones with high enantiomeric purity, which are useful intermediates from which to prepare 5-[(R)-2-(5,6-diethyl-indan-2-ylamino)-1-hydroxy-ethyl]-8-hydroxy-(*1H*)-quinolin-2-one salts.

5-[(R)-2-(5,6-Diethyl-indan-2-ylamino)-1-hydroxy-ethyl]-8-hydroxy-(*1H*)-quinolin-2-one salts are β-selective adrenoceptor agonists with potent bronchodilator activity. For example, 5-[(R)-2-(5,6-diethyl-indan-2-ylamino)-1-hydroxy-ethyl]-8-hydroxy-(*1H*)-quinolin-2-one maleate is especially useful for treating asthma and chronic obstructive pulmonary disease (COPD).

In a first aspect the invention provides a process for preparing 8-substituted oxy-5-((*R*)-2-halo-1-hydroxy-ethyl)-(*1H*)-quinolin-2-ones or acceptable solvates thereof comprising reacting a 5-(α-haloacetyl)-8-substituted oxy-(*1H*)-quinolin-2-one with a reducing agent in the presence of a chiral agent and a base to form a 8-(substituted oxy)-5-((*R*)-2-halo-1-hydroxy-ethyl)-(*1H*)-quinolin-2-one, said chiral agent having a formula I or II 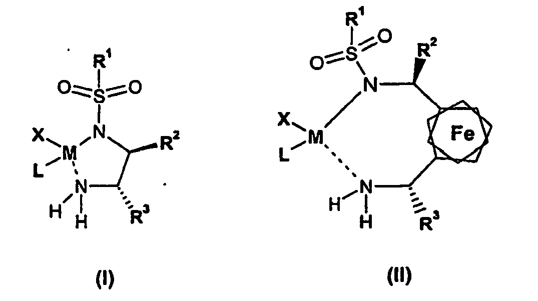 wherein
M is Ru, Rh, Ir, Fe, Co or Ni;
L is C₆-C₂₄-aryl or a C₆-C₂₄.aryl-C₁-C₁₀-aliphatic residue, in either case being optionally linked to a polymer;
X is hydrogen or halo;
R¹ is a C₁-C₁₀-aliphatic, C₃-C₁₀-cycloaliphatic, C₃-C₁₀-cycloaliphatic- C₁-C₁₀-aliphatic, C₆-C₂₄-aryl, C₆-C₂₄-aryl- C₁-C₁₀-aliphatic residue or a 4- to 12-membered heterocyclic group, which, in each case, is optionally linked to a polymer; and
R² and R³ are phenyl,
or R² and R³ together with the carbon atom to which they are attached form a cyclohexane or cyclopentane ring.

This process provides an efficient process for preparing 8-substituted oxy-5-((*R*)-2-halo-1-hydroxy-ethyl)-(*1H*)-quinolin-2-ones, especially 8-phenylmethoxy-5-((*R*)-2-chloro-1-hydroxy-ethyl)-(*1H*)-quinolin-2-one, for large scale production with high enantiomeric purity and yield.

Terms used in the specification have the following meanings:
"Halo" or "halogen" as used herein denotes an element belonging to group 17 (formerly group VII) of the Periodic Table of Elements, which may be, for example, fluorine, chlorine, bromine or iodine. Preferably halo or halogen is chlorine, bromine or iodine.
"C₁-C₁₀-Aliphatic residue or group" as used herein denotes an acyclic, saturated or unsaturated, non-aromatised hydrocarbon group having up to 10 carbon atoms, for example C₁-C₁₀-alkyl, C₂-C₁₀-alkenyl or C₂-C₁₀-alkynyl. Preferably the C₁-C₁₈-aliphatic residue or group is a C₁-C₄-aliphatic residue or group, especially ethyl, propyl or butyl.
"C₃-C₁₀-Cycloaliphatic residue or group" as used herein denotes a cyclic, saturated or unsaturated, non-aromatised hydrocarbon group having 3 to 10 carbon atoms, for example C₃-C₁₀-cycloalkyl or C₃-C₁₀ cycloalkenyl. Preferably the C₃-C₁₀-cycloaliphatic residue or group is a C₃-C₈-cycloaliphatic residue or group, especially C₃-C₁₀-cycloalkyl or C₃-C₁₀-cycloalkenyl.
C₃-C₁₀-Cycloahphatic-C₁-C₁₀-aliphatic residue or group" as used herein denotes a C₁-C₁₀-aliphatic residue or group as hereinbefore defined that is substituted by a C₃-C₁₀-cycloaliphatic residue or group as hereinbefore defined, for example C₃-C₁₀-cycloalkyl-C₁-C₁₀-alkyl, C₃-C₁₀ cycloalkyl-C₂-C₁₀-alkenyl, C₃-C₁₀-cycloalkyl-C₂-C₁₀-alkynyl, C₃-C₁₀-cycloalkenyl-C₁-C₁₀-alkyl, C₃-C₁₀-cycloalkenyl-C₂-C₁₀-alkenyl, C₃-C₁₀-cycloalkenyl-C₂-C₁₀-alkynyl, C₃-C₁₀-_{C}ycloalkynyl-C₁-C₁₀-alkyl, C₃-C₁₀-cycloalkynyl-C₂-C₁₀-alkenyl or C₃-C₁₀-cycloalkynyl-C₂-C₁₀-alkynyl. Preferably the C₃-C₁₀-cycloaliphatic-C₁-C₁₀-aliphatic residue or group is a C₃-C₈-cycloaliphatic-C₁-C₄-aliphatic residue or group, especially cyclopropylmethyl.
"C₆-C₂₄-Aryl residue or group" as used herein denotes aryl having 6 to 24 carbon atoms. The C₆-C₂₄-aryl residue is preferably unsubstituted, however, it may be substituted, for example, by one or more, e.g., two or three, residues, e.g., those selected from halo, C₁-C₁₀-alkyl, halo-C₁-C₁₀-alkyl, C₂-C₁₀-alkenyl, C₁-C₁₀-alkoxy, hydroxy, -CHO, C₁-C₁₀ substituted oxy, C₂-C₁₀-alkanoyl-oxy, phenyl, phenoxy, halo-substituted-phenoxy, amino, C₁-C₁₀-alkylamino, di(C₁-C₁₀-alkyl)amino, nitro, cyano and CF₃. Preferably the C₆-C₂₄-aryl residue or group is a C₆-C₂₀-aryl residue or group, especially phenyl, isopropylmethylbenzene (cymene), benzene, hexamethylbenzene, mesitylene, 4-chloro-4-phenoxy-phenyl, 4-phenoxy-phenyl, 5-dimethylamino-1-naphthyl,5-diethylamino-1-naphthyl, 5-nitro-1-naphthyl, 2-nitrophenyl, 3- nitrophenyl, 4-nitrophenyl, 4-vinylphenyl, 4-biphenylyl, 9-anthracenyl, 2-hydroxyphenyl, 3- hydroxyphenyl, 4-hydroxyphenyl, tolyl, phenanthryl, dimethyl-(naphthalene-1-yl)-amine, mono to tristrifluoromethylphenyl, chrysenyl or perylenyl.
"C₆-C₂₄-Aryl-C₁-C₁₀-aliphatic residue or group" as used herein denotes a C₁-C₁₀-aliphatic residue or group as hereinbefore defined that is substituted by a C₆-C₂₄-aryl residue or.group as hereinbefore defined. Preferably the C₆-C₂₄-aryl-C₁-C₁₀-aliphatic residue or group arylaliphatic residue is a C₆-C₂₀-aryl-C₁-C₄-aliphatic residue or group, especially phenyl-C₁-C₄-alkyl, phenyl-C₂-C₄-alkenyl or phenyl-C₂-C₄-alkynyl.
"C₁-C₁₀-Alkyl" as used herein denotes straight chain or branched alkyl having 1 to 10 carbon atoms. Preferably, C₁-C₁₀-alkyl is C₁-C₄-alkyl.
"C₂-C₁₀-Alkenyl" as used herein denotes straight chain or branched alkenyl having 2 to 10 carbon atoms. Preferably, C₂-C₁₀-alkenyl is C₂-C₄-alkenyl.
"C₂-C₁₀-Alkynyl" as used herein denotes straight chain or branched alkynyl having 2 to 10 carbon atoms. Preferably, C₂-C₁₀-alkynyl is C₂-C₄-alkynyl.
"C₃-C₁₀-Cycloalkyl" as used herein denotes cycloalkyl having 3 to 10 ring carbon atoms, for example cyclopropyl, cyclobutyl, cyclopentyl, cyclohexyl, cycloheptyl or cyclooctyl, any of which can be substituted by one, two or more C₁-C₄-alkyl groups, particularly methyl groups. Preferably, C₃-C₁₀-cycloalkyl is C₃-C₈-cycloalkyl, especially C₃-C₆-cycloalkyl.
"C₃-C₁₀-Cycloalkenyl" as used herein denotes cycloalkenyl having 3- to 10-ring carbon atoms, for example cyclopropenyl, cyclobutenyl, cyclopentenyl, cyclohexenyl, cycloheptenyl or cyclooctenyl, any of which can be substituted by one, two or more C₁-C₄-alkyl groups, particularly methyl groups. Preferably, C₃-C₁₀-cycloalkenyl is C₃-C₈-cycloalkenyl, especially C₃-C₆-cycloalkenyl, in particular, cyclopent-2-en-yl, cyclopent-3-en-yl, cyclohex-2-en-yl or cyclohex-3-en-yl.
"Benzo-C₃-C₁₀-cycloalkyl" as used herein denotes C₃-C₁₀-cycloalkyl as hereinbefore defined attached at two adjacent carbon atoms to a benzene ring. Preferably, benzo-C₃-C₁₀-cycloalkyl is benzo-C₃-C₈-cycloalkyl, especially, benzocyclohexyl (tetrahydronaphthyl). "C₃-C₁₀-Cycloalkyl-C₁-C₁₀-alkyl as used herein denotes C₁-C₁₀-alkyl as hereinbefore defined that is substituted by C₃-C₁₀-cycloalkyl as hereinbefore defined. Preferably, C₃-C₁₀-cycloalkyl-C₁-C₁₀-alkyl cycloalkylalkyl is C₃-C₈-cycloalkyl-C₁-C₄-alkyl.
"C₇-C₃₄-Aralkyl" as used herein denotes straight-chain or branched C₆-C₂₄-aryl-C₁-C₁₀-alkyl. and may be, e.g., one of the C₁-C₁₀-alkyl groups mentioned hereinbefore, particularly one of the C₁-C₄-alkyl groups, substituted by phenyl, tolyl, xylyl or naphthyl. Preferably, C₇-C₃₄-aralkyl is C₇-C₁₄-aralkyl, especially phenyl-C₁-C₄-alkyl, particularly benzyl or 2-phenylethyl.
"C₁-C₁₀-Alkoxy" as used herein denotes straight chain or branched alkoxy having 1 to 10 carbon atoms. Preferably, C₁-C₁₀-alkoxy is C₁-C₄-alkoxy.
"4- to 12-membered heterocyclic group" as used herein denotes a monovalent heterocyclic group having 4 to 12 carbon atoms and one, two, three or four heteroatoms selected from nitrogen, oxygen and sulfur. The 4- to 12-membered heterocyclic group may be, for example, a monocyclic ring with one nitrogen, oxygen or sulfur atom, such as azetidinyl, pyrryl, pyridyl, piperidyl, pyranyl, furyl, tetrahydrofuryl or thienyl, a monocyclic ring with two hetero atoms selected from nitrogen, oxygen and sulfur, such as imidazolyl, pyrimidinyl, piperazinyl, oxazolyl, isoxazolyl, thiazolyl, morpholinyl or thiomorpholinyl, or a bicyclic ring such as benzazole, indole, benzimidazole, indazole, benzothiophene, benzothiazole or benzodioxole. The 4- to 12-membered heterocyclic group can be an unsubstituted or substituted. Preferred substituents on the heterocyclic ring include halo, cyano, hydroxy, carboxy, aminocarbonyl, nitro, C₁-C₁₀-alkyl, hydroxy-C₁-C₄-alkyl, C₁-C₁₀-alkoxy, C₃-C₁₀-cycloalkyl, C₁-C₄-alkylcarbonyl and phenyl-C₁-C₄-alkyl. Preferably, the 4- to 12-membered heterocyclic group is a 5- to 8-membered heterocyclic group, especially a monocyclic ring having one or two nitrogen or oxygen atoms such as pyranyl or 2-, 3- or 4-pyridyl, or one nitrogen atom and one oxygen atom, in the ring and optionally substituted on a ring nitrogen atom by C₁-C₄-alkyl, hydroxy-C₁-C₄-alkyl, C₁-C₄-alkylcarbonyl or phenyl-C₁-C₄-alkyl, or a bicyclic ring such as benzo[1,3]-dioxole.
"Halo-C₁-C₁₀-alkyl" as used herein denotes straight-chain or branched alkyl as hereinbefore defined that is substituted by one or more, e.g., one, two or three, halogen atoms as hereinbefore defined. Preferably, halo-C₁-C₁₀-alkyl is halo-C₁-C₄-alkyl, especially where halo is fluorine or chlorine.
"Substituted silyl group" as used herein denotes is preferably a silyl group substituted with at least one C₁-C₁₀-alkyl group as herein defined.

Throughout this specification and in the claims that follow, unless the context requires otherwise, the word "comprise", or variations such as "comprises" or "comprising", will be understood to imply the inclusion of a stated integer or step or group of integers or steps but not the exclusion of any other integer or step or group of integers or steps.

In a preferred embodiment of the process for preparing 8-substituted oxy-5-((*R*)-2-halo-1-hydroxy-ethyl)-(1*H*)-quinolin-2-ones or acceptable solvates thereof the chiral agent has formula I or II as hereinbefore defined, wherein
M is ruthenium;
L is isopropylmethylbenzene, benzene, hexamethylbenzene or mesitylene;
X is hydrogen or halo, preferably chloro;
R¹ is phenyl, 2- or 3- or 4-pyridyl, 4'-chloro-4-phenoxy-phenyl, 4-phenoxy-phenyl, 5-dimethylamino-1-naphthyl, 5-nitro-1-naphthyl, 2-, 3-, 4-nitrophenyl, 4-vinylphenyl, 4-biphenylyl, 9-anthracenyl, 2-, 3- or 4-hydroxyphenyl, tolyl, phenanthryl, benzo[1,3]-dioxole, dimethyl(naphthalene-1-yl)-amine, mono to tristrifluoromethylphenyl, chrysenyl, perylenyl or pyranyl; and
R² and R³ are both phenyl.

In a particularly preferred embodiment of the process for preparing 8-substituted oxy-5-((*R*)-2-halo-1-hydroxy-ethyl)-(1*H*-quinolin-2-ones or acceptable solvates thereof the chiral agent is a ruthenium based chiral agent, especially RuCl[(1*S*,2*S*)-*p*-TsN-CH(C₆H₅)CH(C₆H₅)-NH₂](η⁶-*p*-cymene).

In a second aspect the invention provides a process for preparing 5-[(R)-2-(5,6-diethyl-indan-2-ylamino)-1-hydroxy-ethyl]-8-hydroxy-(1*H*)-quinolin-2-one salts comprising:
(i) reacting a 5-(α-haloacetyl)-8-substituted oxy-(1*H*-quinolin-2-one with a reducing agent in the presence of a chiral agent and a base to form a 8-substituted oxy-5-((*R*)-2-halo-1-hydroxy-ethyl)-(*1H*)-quinolin-2-one, said chiral agent having a formula I or II as hereinbefore defined;
(ii) treating the 8-substituted oxy-5-((*R*)-2-halo-1-hydroxy-ethyl)-(*1H*)-quinolin-2-one with a base in the presence of a solvent to form a 8-substituted oxy-5-(*R*)-oxiranyl-(1*H*)-quinolin-2-one of formula III 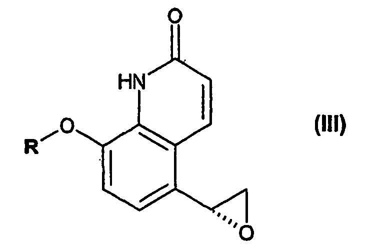 wherein R is a protecting group;
(iii) reacting the 8-substituted oxy-*S*-(R)-oxiranyl-(*1H*)-quinolin-2-one of formula III where R is as hereinbefore defined, with 2-amino-(5-6-diethyl)-indan to form a reaction mixture containing compounds having formulae IV, V and VI 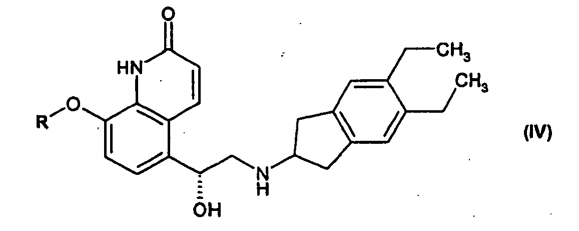 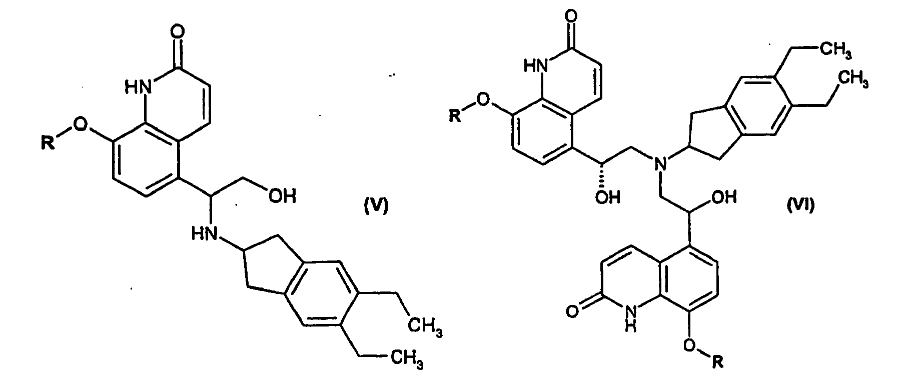 wherein R is a protecting group;
(iv) treating the reaction mixture prepared in Step (iii) with an acid in the presence of a solvent to form a corresponding salt;
(v) isolating and crystallizing a salt having formula VII 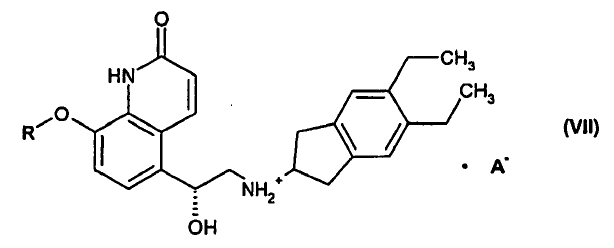 wherein R is a protecting group and A is an anion;
(vi) removing the protecting group from the salt having formula VII in the presence of a solvent to form a salt having Formula VIII 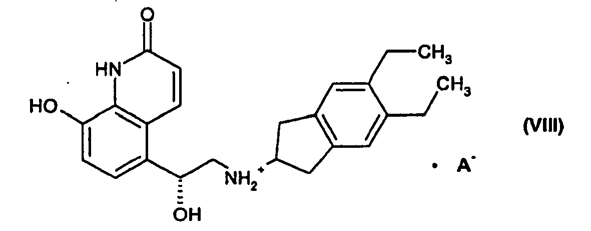 wherein A- is an anion; and
(vii) treating the salt having formula VIII with an acid in the presence of a solvent to form 5-[(R)-2-(5,6-diethyl-indan-2-ylamino)-1-hydroxy-ethyl]-8-hydroxy-(1*H*)-quinolin-2-one salt having formula IX 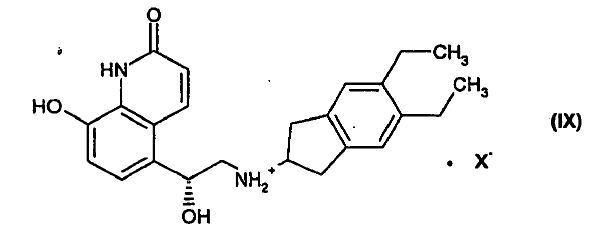 wherein X is an anion.

In a third aspect the invention provides a process for preparing 5-[(R)-2-(5,6-diethyl-indan-2-ylamino)-1-hydroxy-ethyl]-8-hydroxy-(*1H*)-quinolin-2-one salts comprising:
(a) reacting
   (i) 8-hydroxy-(*1H*)-quinolin-2-one with an acylating agent and a Lewis acid to form 5-acetyl-8-hydroxy-(*1H*)-quinolin-2-one; or
   (ii) 8-hydroxy-(*1H*)-quinolin-2-one with an acylating agent to form 8-acetoxy-(*1H*)-quinolin-2-one, and treating, in-situ, the 8-acetoxy-(*1H*)-quinolin-2-one with a Lewis acid to form 5-acetyl-8-hydroxy-(*1H*)-quinolin-2-one; or
   (iii) 8-acetoxy-(*1H*)-quinolin-2-one with a Lewis acid to form 5-acetyl-8-hydroxy-(1*H*)-quinolin-2-one;
(b) reacting the 5-acetyl-8-hydroxy-(*1H*)-quinolin-2-one prepared in Step (a) with a compound having the formula R-Q in the presence of a base and a solvent to form 5-acetyl-8-substituted oxy-(*1H*)-quinolin-2-one, wherein R is a protecting group and Q is a leaving group;
(c) reacting the 5-acetyl-8-substituted oxy-(*1H*)-quinolin-2-one with a halogenating agent in the presence of a solvent to form a 5-(α-haloacetyl)-8-substituted oxy-(*1H*)-quinolin-2-one;
(d) reacting the 5-(α-haloacetyl)-8-substituted oxy-(*1H*)-quinolin-2-one with a reducing agent in the presence of a chiral agent and a base to form 8-substituted oxy-5-((*R*)-2-halo-1-hydroxy-ethyl)-(*1H*)-quinolin-2-one, said chiral agent having a formula I or II as hereinbefore defined;
(e) treating the 8-substituted oxy-5-((*R*)-2-halo-1-hydroxy-ethyl)-(*1H*)-quinolin-2-one with a base in the presence of a solvent to form a 8-substituted oxy-5-(*R*)-oxiranyl-(*1H*)-quinolin-2-one of formula III 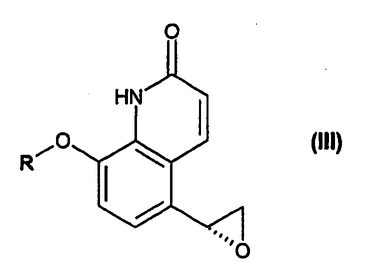 wherein R is a protecting group;
(f) reacting the 8-substituted oxy-5-(R)-oxiranyl-(*1H*)-quinolin-2-one of formula III where R is as hereinbefore defined, with 2-amino-(5-6-diethyl)-indan to form a reaction mixture containing compounds having formulae IV, V and VI 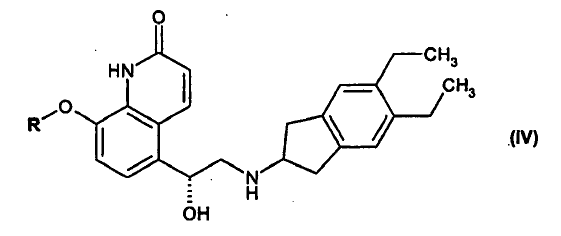 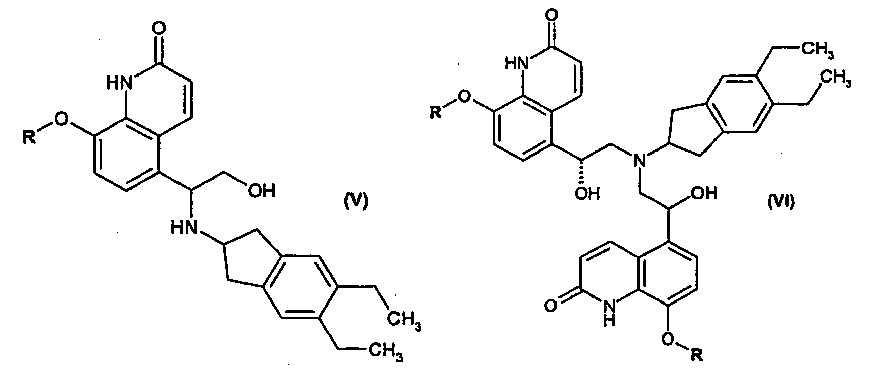 wherein R is a protecting group;
(g) treating the reaction mixture prepared in Step (f) with an acid in the presence of a solvent to form a corresponding salt;
(h) isolating and crystallizing a salt having formula VII 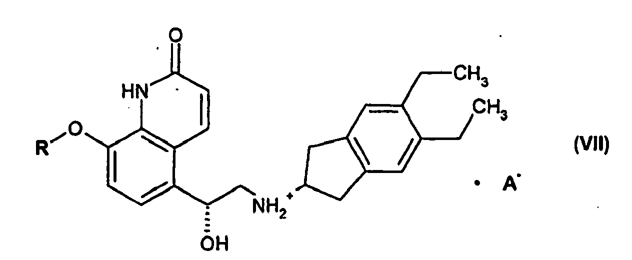 wherein R is a protecting group and A is an anion;
(i) removing the protecting group from the salt having formula VII in the presence of a solvent to form a salt having Formula VIII 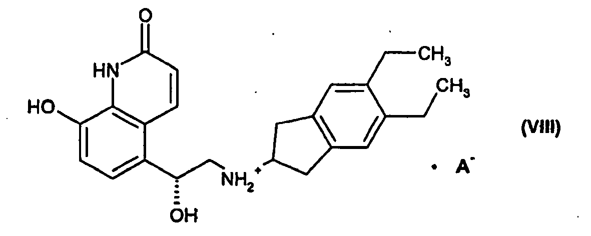 wherein A is an anion; and
(j) treating the salt having formula VIII with an acid in the presence of a solvent to a form 5-[(R)-2-(5,6-diethyl-indan-2-ylamino)-1-hydroxy-ethyl]-8-hydroxy-(*1H*)-quinolin-2-one salt having formula IX 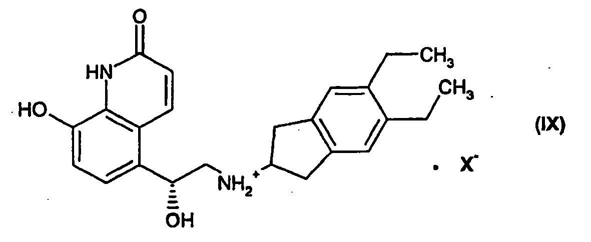 wherein X- is an anion.

In a first aspect the present invention provides a process for preparing 8-substituted oxy-5-((*R*)-2-halo-1-hydroxy-ethyl)-(*1H*)-quinolin-2-ones or acceptable solvates thereof comprising reacting a 5-(α-haloacetyl)-8-substituted oxy-(*1H*-quinolin-2-one with a reducing agent in the presence of a chiral agent of formula I or II and a base to form a 8-substituted oxy-5-((*R*)-2-halo-1-hydroxy-ethyl)-(*1H*)-quinolin-2-one.

The 5-(α-haloacetyl)-8-substituted oxy-(*1H*)-quinolin-2-one has formula X 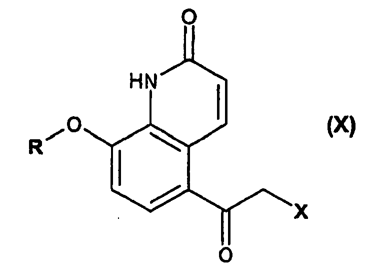 wherein R is a protecting group; and X is a halogen. The halogen is selected from bromine, chlorine, fluorine and iodine. Preferably, the halogen is chlorine.

The 8-substituted oxy-5-((*R*)-2-halo-1-hydroxy-ethyl)-(*1H*)-quinolin-2-one has formula XI 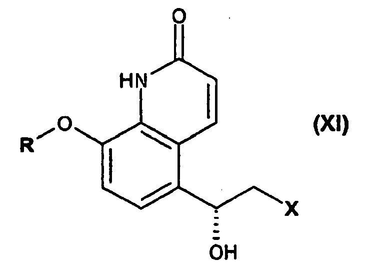 wherein R is a protecting group; and X is a halogen. The halogen is selected from bromine, chlorine, fluorine and iodine. Preferably, the halogen is chlorine.

The chiral agent is a compound of formula I or II as hereinbefore defined.

M is ruthenium, rhodium, iridium, iron, cobalt or nickel, but it is preferably ruthenium.

L is preferably isopropylmethylbenzene, benzene, hexamethylbenzene or mesitylene, but especially isopropylmethylbenzene. L is optionally linked to a polymer. Suitable polymers include polystyrene (PS), cross-linked PS (J), polyethylene glycol (PEG) or a silica gel residue (Si). Examples are NH-R⁴, wherein R⁴ is C(O)(CH₂)ₙ-PS or C(O)NH(CH₂)ₙ-PS; and -O-Si(R⁵)₂(CH₂)R⁶, wherein n is 1-7, R⁵ is C₁-C₆ alkyl, e.g., ethyl, and R⁶ is a polystyrene, cross-linked polystyrene, polyethylene glycol or a silica gel residue.

X is hydrogen or halo. It is preferably halo, especially chloro.

R¹ is preferably phenyl, 2- or 3- or 4-pyridyl, 4'-chloro-4-phenoxy-phenyl, 4-phenoxy-phenyl, 5-dimethylamino-1-naphthyl, 5-nitro-1-naphthyl, 2-, 3-, 4-nitrophenyl, 4-vinylphenyl, 4-biphenylyl, 9-anthracenyl, 2-, 3- or 4-hydroxyphenyl, tolyl, phenanthryl, benzo[1,3]-dioxole, dimethyl(naphthalene-1-yl)-amine, mono to tristrifluoromethylphenyl, chrysenyl, perylenyl or pyrenyl.

R¹ is optionally linked to a polymer. Suitable polymers include polystyrene (PS), cross-linked PS (J), polyethylene glycol (PEG) or a silica gel residue (Si). Examples are NH-R⁴, wherein R⁴ is C(O)(CH₂)ₙ-PS or C(O)NH(CH₂)ₙ-PS; and -O-Si(R⁵)₂(CH₂)ₙR⁶, wherein n is 1-7, R⁵ is C₁-C₆ alkyl, e.g., ethyl, and R⁶ is a polystyrene, cross-linked polystyrene, polyethylene glycol or a silica gel residue.

R² and R³ are preferably both phenyl.

Chiral agents of formula I and their use in asymmetric hydrogen transfer reactions between alcohols or formic acid and ketones are described in K. Haack et al Agnew. Chem. Int. Ed. Engl. 1997, Vol 36, no.3, pages 285-288.

The chiral agent of formula I reacts with a base, such as potassium hydroxide or triethylamine, in a solvent such as CH₂Cl₂, methanol, dimethylformamide, or dimethylacetamide or a mixture of methanol and dimethylformamide or a mixture of methanol and dimethylacetamide, and upon elimination of a hydrogen halide forms a compound of formula XIII 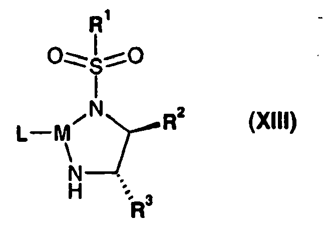 wherein M, L, R', R² and R³ are as hereinbefore defined. The compound of formula XIII reacts with a reducing agent to form the compound of formula I where X is hydrogen.

Preferably the process of the present invention is carried out by adding a chiral agent of formula I as hereinbefore defined where X is halo to the 5-(a-haloacetyl)-8-substituted oxy-(*1H*-quinolin-2-one and the reducing agent in the presence|of a base such as potassium hydroxide or triethylamine in a solvent such as a mixture of methanol and dimethylformamide or a mixture of methanol and dimethylacetamide. The base converts the chiral agent of formula I where X is halo to the compound of formula XIII which, itself, reacts with the reducing agent to form the chiral agent of formula I where X is hydrogen. As an alternative, the compound of formula I where X is halo is formed *in situ* by adding a metal-halide dimer such as[RuCl₂(p-cymene)]₂ and a chiral ligand such as (1S,2S)-(+)-N-p-tosyl-1,2-diphenylethylendiamine separately.

The process of the present invention may also be carried out by adding a chiral agent of formula II to the 5-(α-haloacetyl)-8-substituted oxy-(*1H*)-quinolin-2-one and the reducing agent in the presence of a base.

Chiral agents of formula II include those that are described in Puentener et al Tetrahedron Letters, 1996, Vol 37, no.45 pages 8165-8168. The chiral agent of formula II reacts with a base, such as potassium hydroxide or triethylamine, in a solvent such as CH₂Cl₂, methanol, dimethylformamide or dimethylacetamide or a mixture of methanol and dimethylformamide or a mixture of methanol and dimethylacetamide, and upon elimination of a hydrogen halide forms a compound of formula XV 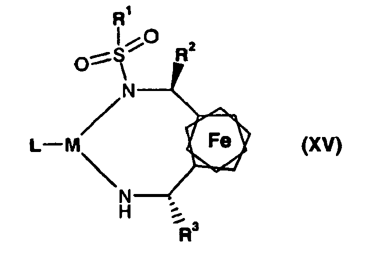 wherein M, L, R² and R³ are as hereinbefore defined. The compound of formula XV reacts with a reducing agent to form the chiral agent of formula II as hereinbefore defined where X is hydrogen.

The process of the present invention may be carried out by adding a pre-prepared chiral agent of formula II to the 5-(α-haloacetyl)-8-substituted oxy-(*1H*)-quinolin-2-one and the reducing agent in the presence of a base. For example, a chiral agent of formula II where X is halo is added to the 5-(α-haloacetyl)-8-substituted oxy-(*1H*)-quinolin-2-one and the reducing agent in the presence of a base and a solution of potassium hydroxide in a solvent. The base converts the chiral agent of formula II where X is halo to the compound of formula XV which, itself, reacts with the reducing agent to form the chiral agent of formula I where X is hydrogen.As an alternative, the compound of formula II where X is halo is formed *in situ* by adding a metal-halide dimer and a chiral ligand separately.

The chiral agent is preferably a pre-prepared compound of formula I, especially a compound of formula XVI 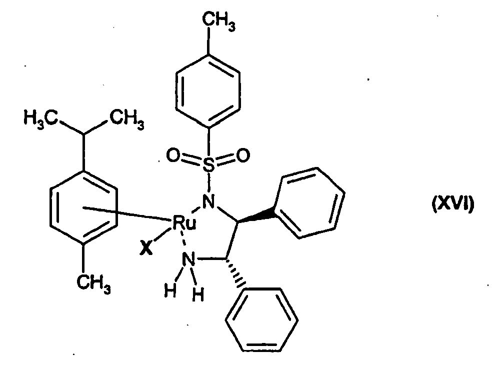 where X is hydrogen or halo. Preferably the chiral agent is RuCl[(1*S*,2*S*)-*p*-TsN-CH(C₆H₅)CH(C₆H₅)-NH₂](η⁶-*p*-cymene).

Alternatively, the chiral agent is a compound of formula I where X is halo that is formed *in situ* by adding the metal-halide dimer and the chiral ligand separately. For example, RuCl[(1*S*,2*S*)-*p*-TsN-CH(C₆H₅)CH(C₆H₅)-NH₂](η⁶-*p*-cymene) can be formed by reacting the RuCl₂ dimer, [Ru(η⁶-*p*-cymene)Cl₂]₂, together with the chiral ligand, *S,S*-TsDPEN ((1*S*,2*S*)-*p-*TsNH-CH(C₆H₅)CH(C₆H₅)-NH₂), *in situ* to give Rucl[(1*S*,2*S*)-*p*-TsN-CH(C₆H₅)CH(C₆H₅)-NH₂](η⁶-*p*-cymene), which has formula XVII

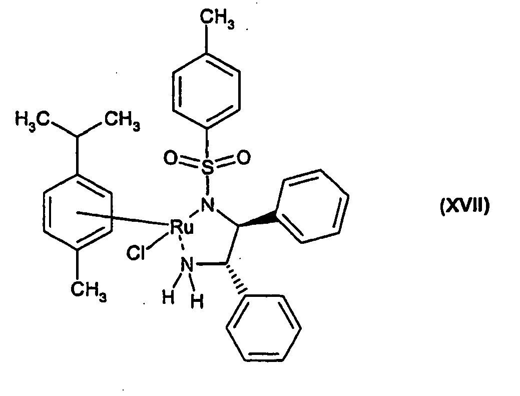

using the procedure described in K. Haack et al Agnew. Chem. Int. Ed. Engl. 1997, Vol 36, no.3, pages 285-288.

Suitable reducing agents include formic acid, primary alcohols and secondary alcohols. Preferred reducing agents include formic acid, 2-propanol and 3-pentanol.

When the chiral agent is a ruthenium based agent, the reducing agent is preferably 2-propanol, 3-pentanol or formic acid. More preferably, the formic acid is used in the presence of an amine, most preferably a tertiary amine such as triethylamine, tributyl amine, 2,2,6,6-tetramethylpiperidine, 1,2,2,6,6-pentamethylpiperidine and N,N-diisopropylethylamine. The reducing agent may also be used as a solvent, especially 2-propanol and most preferably formic acid.

The amount of chiral agent is preferably between about 0.1 to about 10 mole %, especially between about 0.8 and 1 mole %, referring to the compound of formula X.

The reaction is carried out in the presence of a base. The temperature used is preferably from about -10 °C to about 80 °C, but especially from about 0 °C to about 50 °C.

When the reducing agent is formic acid the base is preferably a tertiary amine, for example triethylamine. Triethylamine is preferably used in molar excess to formic acid as this significantly accelerates this reaction. This allows the reaction to be performed at a lower temperature, for example from about 25 °C to about 50°C, but preferably about 30°C. This also provides for better enantioselectivities i.e. more of the R isomer of compound of formula X is produced and less of the S isomer of that compound is produced. Preferably the molar ratio of triethylamine to formic acid is from 1:1 to 2:5, but especially about 1:2. When the reducing agent is an alcohol the base is preferably potassium hydroxide or sodium hydroxide.

A solvent is preferably used. The solvent is preferably an alkyl acetate, e.g. a C₁-C₆-alkyl acetate such as ethyl acetate, isopropyl acetate or butyl acetate, a lower alkyl alcohol, e.g. a C₁-C₆-alkyl alcohol such as methanol, ethanol, propanol, isopropanol, butanol or pentanol; an aliphatic C₁-C₁₂-hydro-carbon such as isooctane, heptane; dimethylformamide; dimethylacetamide; an aromatic hydrocarbon such as toluene or benzene; acetonitrile; a heterocycle such as tetrahydrofuran; a dialkyl ether such diisopropyl ether, 2-methoxyethyl ether or diethylene ether; an aqueous solvent such as water; an ionic liquid; or a chlorinated solvent such as methylenechloride. A combination of solvents may also be used. When the chiral agent is a ruthenium based agent the solvent is preferably methanol, methylene-chloride, dimethylformamide or dimethylacetamide. However a combination of methanol and dimethylformamide or a combination of methanol and dimethylacetamide is especially preferred, for example using 90 volumes of methanol with 10 volumes dimethylformamide/ dimethylacetamide.

Preferably the 5-(α-haloacetyl)-8-substituted oxy-(*1H*)-quinolin-2-one is reacted with formic acid in the presence of a chiral ruthenium agent and a tertiary amine to form the 8-substituted oxy-5-((*R*)-2-halo-1-hydroxy-ethyl)-(*1H*)-quinolin-2-one. The 8-substituted oxy-5-((*R*)-2-halo-1-hydroxy-ethyl)-(*1H*)-quinolin-2-one of formula XI is preferably 8-phenyl-methoxy-5-((*R*)-2-chloro-1-hydroxy-ethyl)-(*1H*)-quinclin-2-one.

The 8-substituted oxy-5-((*R*)-2-halo-1-hydroxy-ethyl)-(*1H*)-quinolin-2-one product is optionally purified by any of the various techniques known to the art, for example by crystallization, and optionally in the presence of charcoal.

As mentioned above, the 8-substituted oxy-5-((R)-2-halo-1-hydroxy-ethyl)-(*1H*)-quinolin-2-ones that are prepared from 5-(α-haloacetyl)-8-substituted oxy-(*1H*)-quinolin-2-ones in accordance with the first aspect of the present invention may be used to prepare 5-[(R)-2-(5,6-diethyl-indan-2-ylamino)-1-hydroxy-ethyl]-8-hydroxy-(*1H*)-quinolin-2-one salts. The second aspect of the present invention involves reacting a 5-(α-haloaceryl)-8-substituted oxy-(*1H*)-quinolin-2-one with a reducing agent in the presence of a chiral agent and a base to form an 8-substituted oxy-5-((*R*)-2-halo-1-hydroxy-ethyl)-(*1H*)-quinolin-2-one (step i), and its subsequent conversion to a 5-[(R)-2-(5,6-diethyl-indan-2-ylamino)-1-hydroxy-ethyl]-8-hydroxy-(*1H*)-quinolin-2-one salt (steps ii through vii).

Step (i) is carried out as described above in connection with the first aspect of the present invention.

In step (ii) the 8-substituted oxy-5-((*R*)-2-halo-1-hydroxy-ethyl)-(*1H*)-quinolin-2-one formed in step (i) is converted to an 8-substituted oxy-5-(*R*)-oxiranyl-(*1H*)-quinolin-2-one.

In a preferred embodiment of the invention, the 5-(α-haloacetyl)-8-substituted oxy-(*1H*)-quinolin-2-one is reacted with the reducing agent in the presence of the chiral agent and a base to form the 8-substituted oxy-5-(*R*)-oxiranyl-(*1H*)-quinolin-2-one in a single step i.e. steps (i) and (ii) are combined. The base is preferably potassium t-butoxide, potassium hydroxide or potassium isopropoxide.

Alternatively, the chiral agent is prepared *in situ*, for example by adding [Ru(η⁶-*p-*cymene)Cl₂]₂ together with a chiral ligand, such as *S,S*-TsDPEN ((1*S*,2*S*)-*p*-TsNH-CH(C₆H₅)CH(C₆H₅)-NH₂) to give RuCl[(1*S*,2*S*)*-p-*TsN-CH(C₆H₅)CH(C₆H₅)-NH₂](η⁶*-p-*cymene), which is converted upon addition of a base such as potassium hydroxide or triethylamine, to give RuH[(1*S*,2*S*)*-p-*TsN-CH(C₆H₅)CH(C₆H₅)-NH₂](η⁶*-p-*cymene).

The base used in step (ii) is preferably ethoxide, sodium hydroxide, potassium phosphate, potassium carbonate, potassium hydrogencarbonate or caesium carbonate, but especially potassium carbonate. A combination of bases may also be used.

The solvent used in Step (ii) is preferably an alkyl acetate, e.g. a C₁₋C₆-alkyl acetate such as ethyl acetate, isopropyl acetate or butyl acetate; a lower alkyl alcohol, e.g. a C₁₋C₆-alkyl alcohol such as methanol, ethanol, propanol, isopropanol, butanol or pentanol; an aliphatic C₁-C₁₂-hydrocarbon such as isooctane, heptane; dimethylformamide; an aromatic hydrocarbon such as toluene or benzene; a dialkyl ketone such as acetone, ethyl methylketone (2-butanone) or methyl isobutyl ketone; acetonitrile; a heterocycle such as tetrahydrofuran; a dialkyl ether such diisopropyl ether, 2-methoxyethyl ether or diethylene ether; an aqueous solvent such as water; an ionic liquid; or a chlorinated solvent such as methylenechloride. A combination of solvents may also be used. A preferred solvent for use in Step (ii) is a combination of acetone and water, however a combination of 2-butanone and water is especially preferred.

The temperature used in Step (ii) is preferably from about 10 °C to about 160 °C. More preferably, the temperature is from about 30 °C to about 90 °C, but especially from about 50 °C to about 80 °C.

The 8-substituted oxy-5-(*R*)-oxiranyl-(*1H*)-quinolin-2-one is preferably 8-phenlymethoxy-5-(*R*)-oxiranyl-(*1H*)-quinolin-2-one.

The 8-substituted oxy-5-(*R*)-oxiranyl-(*1H*)-quinolin-2-one product is optionally purified by any of the various techniques known to the art, for example by crystallization.

Crystallization from toluene or acetone is especially preferred, and is optionally conducted in the presence of charcoal.

In Step (iii) 8-substituted oxy-5-(R)-oxiranyl-(*1H*)-quinolin-2-one having formula III 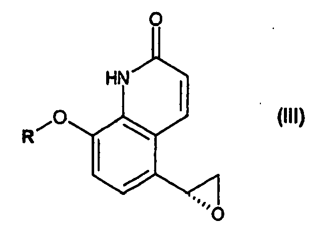 where R is a protecting group, is reacted with 2-amino-(5-6-diethyl)-indan to form a reaction mixture containing compounds having formulae IV, V and VI 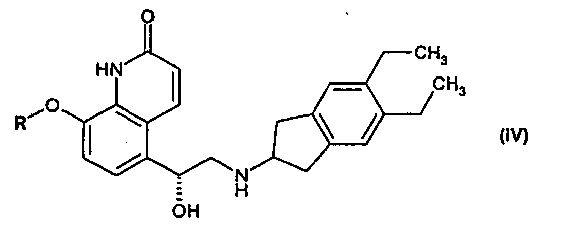 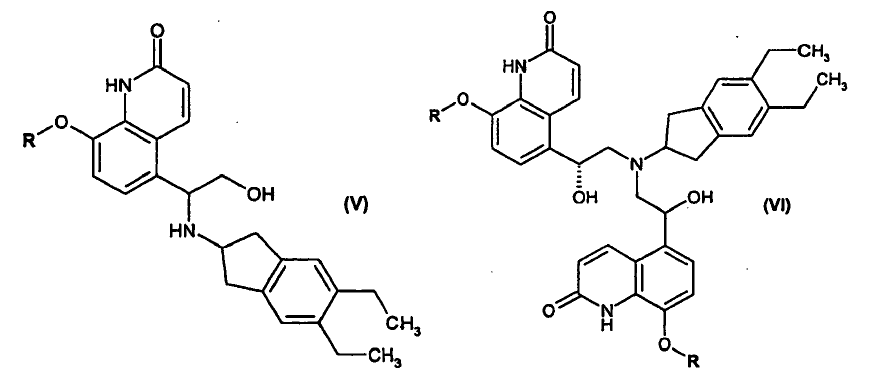 wherein R is a protecting group;

Preferred protecting groups are phenol protecting groups which are known to those skilled in the art. More preferably, the protecting group is selected from the group consisting of alkyl, aryl, alkoxy, alkenyl, cycloalkyl, benzocycloalkyl, cycloalkylalkyl, aralkyl, heterocyclic, heteroaralkyl, haloalkyl, and a substituted silyl group. Most preferably, the protecting group is benzyl or *t*-butyldimethylsilyl.

Preferably, Step (iii) is conducted in the presence of a solvent. Preferred solvents include: alcohols, e.g., C₁₋₆alkyl alcohols, such as methanol, ethanol, propanol, butanol, and pentanol; aliphatic C₆₋₁₂hydrocarbons, e.g., isooctane, heptane; dimethylformamide; dimethylacetamide; aromatic hydrocarbons, such as toluene and benzene; acetonitrile; heterocycles, such as tetrahydro-furan; dialkyl ethers, e.g., diisopropyl ether, 2-methoxyethyl ether and diethylene ether; dimethyl sulfoxide; tetrahydrothiophene 1,1-dioxide, also known as tetramethylene sulfone or as tetramethylene sulfolane; dialkyl carbonate, e.g., dimethyl carbonate and diethyl carbonate; aqueous solvents, such as water; ionic liquids; and chlorinated solvents, such as methylenechloride. A combination of solvents may also be used. More preferably, the solvent is 2-methoxyethyl ether or butanol.

The temperature used in Step (iii) is preferably from about 10 °C to about 160 °C. More preferably, the temperature is from about 30 °C to about 120 °C; and most preferably from about 90 °C to about 120 °C.

Preferably, Step (iii) is conducted with a molar excess of the 2-amino-(5-6-dimethyl)-indan with respect to the 8-substituted oxy-5-(R)-oxiranyl-(*1H*)-quinolin-2-one. Preferably, 1.05 mole equivalent to 3 mole equivalents of 2-amino-(5-6-diethyl)-indan is used with respect to 8-substituted oxy-5-(R)-oxiranyl-(*1H*)-quinolin-2-one. Most preferably, 1.1 mole equivalents to 1.5 mole equivalents of 2-amino-(5-6-diethyl)-indan is used with respect to 8-substituted oxy-5-(R)-oxiranyl-(*1H*)-quinolin-2-one.

The 8-substituted oxy-5-(R)-oxiranyl-(*1H*)-quinolin-2-one is preferably 8-phenylmethoxy-5-(R)-oxiranyl-(*1H*)-quinolin-2-one. The 5-[(R)-2-(5,6-diethyl-indan-2-ylamino)-1-hydroxyethyl]-8-substituted oxy-(*1H*)-quinolin-2-one is preferably 5-[(R)-2-(5,6-diethyl-indan-2-ylamino)-1-hydroxy-ethyl]-8-phenylmethoxy-(*1H*)-quinolin-2-one.

In Step (iv) the reaction mixture prepared in Step (iii) is treated with an acid in the presence of a solvent to form a corresponding salt.

Preferred solvents for use in Step (iv) include: alcohols, e.g. C₁-C₆-alkyl alcohols, such as methanol, ethanol, propanol, butanol, and pentanol; aliphatic C₆-C₁₂-hydrocarbons, e.g., isooctane, heptane; dimethylformamide; dimethylacetamide; aromatic hydrocarbons, such as toluene and benzene; acetonitrile; heterocycles, such as tetrahydrofuran; dialkyl ethers, e.g., diisopropyl ether, 2-methoxyethyl ether and diethylene ether; dimethyl sulfoxide; tetrahydrothiophene 1,1-dioxide, also known as tetramethylene sulfone or as tetramethylene sulfolane; dialkyl carbonate, e.g., dimethyl carbonate and diethyl carbonate; aqueous solvents, such as water; ionic liquids; and chlorinated solvents, such as methylenechloride. A combination of solvents may also be used. More preferably, the solvent is ethanol.

The temperature used in Step (iv) is preferably from about -10 °C to about 160 °C. More preferably, the temperature is from about 0 °C to about 120 °C; and most preferably from about 0 °C to about 75 °C.

In Step (v) a salt having Formula VII 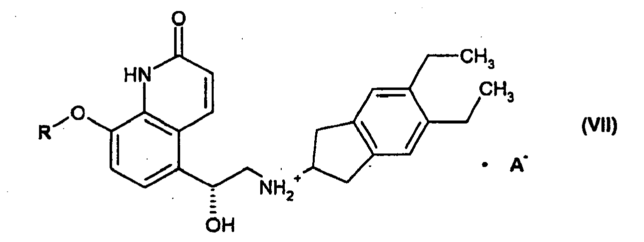 is isolated and crystallized, wherein R is a protecting group; and A- is an anion. The anion corresponds to the acid used in Step (iv). The acid used in Step (iv) is preferably a carboxylic acid, such as benzoic acid, maleic acid, succinic acid, fumaric acid, or tartaric acid; or a mineral acid, such as hydrochloric acid. Most preferably, the acid used in Step (iv) is benzoic acid.

The salt having Formula VII is preferably a benzoate salt having formula XIX 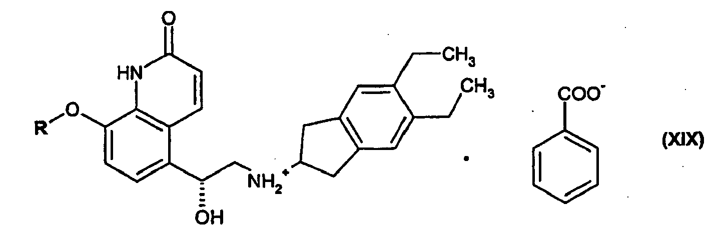 wherein R is a protecting group.

More preferably the benzoate salt of formula XIX is a benzoate salt having formula XX

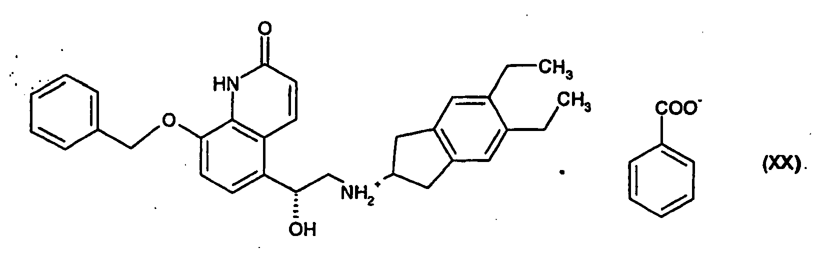

In Step (vi) the protecting group on the salt having formula VII is removed in the presence of a solvent to form a salt having formula VIII 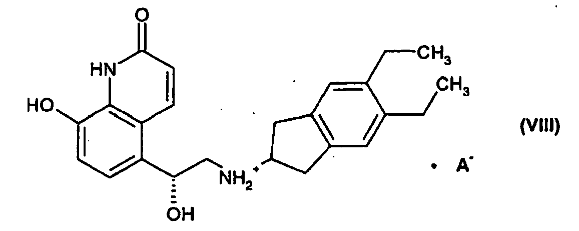 wherein A- is an anion.

The salt having formula VIII is preferably a benzoate salt having formula XXI 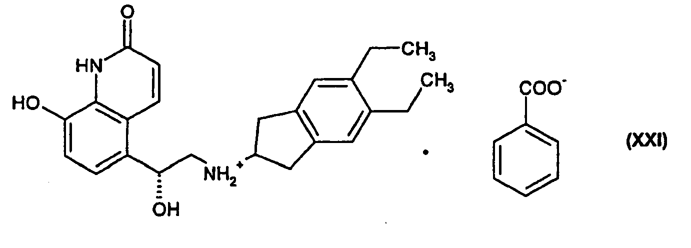 The removal of a protecting group is known to those skilled in the art and depends on the type of protecting group. In one embodiment where the protecting group is benzyl, a preferred method of removing the benzyl group on the salt having formula VII is by treating the salt with hydrogen in the presence of a catalyst. Preferred catalysts include palladium, palladium hydroxide, palladium on activated carbon, palladium on alumina, palladium on carbon powder, platinum, platinum on activated carbon and Raney^{™} nickel. A combination of catalysts may also be used. Most preferably, the catalyst is palladium on activated carbon.

In one embodiment where the protecting group is t-butyldimethylsilyl, a preferred method of removing the *t*-butyldimethylsilyl group on the salt having formula VII is by treating the salt with *t*-butylammonium fluoride or potassium fluoride.

The solvent used in Step (vi) is preferably selected from an alkyl acetate, e.g., C₁-C₆-alkyl acetates, such as ethyl acetate, isopropyl acetate and butyl acetate; lower alkyl alkylamines, e.g., C₁-C₆-alkylamines; alcohols, e.g., C₁-C₆-alkyl alcohols, such as methanol, ethanol, propanol, butanol and pentanol; aliphatic C₆₋C₁₂-hydrocarbons, e.g., isooctane, heptane, dimethylformamide; dimethylacetamide; aromatic hydrocarbons, such as toluene and benzene; acetonitrile; heterocycles, such as tetrahydrofuran; dialkyl ethers, e.g., diisopropyl ether, 2-methoxyethyl ether, and diethylene ether; an acid, e.g., acetic acid, trifluoroacetic acid, and propionic acid; aqueous solvents, such as water; ionic liquids; and chlorinated solvents, such as methylenechloride. A combination of solvents may also be used. More preferably, the solvent is acetic acid or 2-propanol.

The temperature used in Step (vi) is preferably from about 0 °C to about 70 °C. More preferably, the temperature is from about 10 °C to about 50 °C; and most preferably from about 10 °C to about 30 °C.

The salt having formula VIII is preferably 5-[(R)-2-(5,6-diethyl-indan-2-ylamino)-1-hydroxyethyl]-8-hydroxy-(*1H*)-quinolin-2-one benzoate.

In Step (vii) the salt having formula VIII is treated with an acid in the presence of a solvent to form a salt having Formula IX 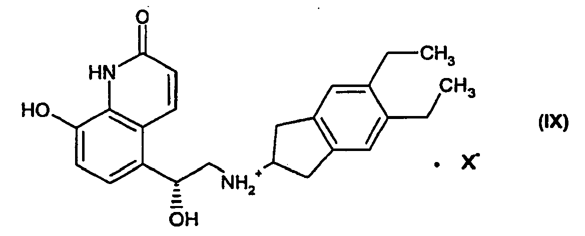 wherein X- is an anion. The anion corresponds to the acid used in Step (vii). The acid used in Step (vii) is preferably a carboxylic acid, such as benzoic acid, maleic acid, succinic acid, fumaric acid, or tartaric acid. Most preferably, the acid used in Step (vii) is maleic acid.

The salt having formula IX is preferably 5-[(R)-2-(5,6-diethyl-indan-2-ylamino)-1-hydroxyethyl]-8-hydroxy-(*1H*)-quinolin-2-one maleate having formula XXII

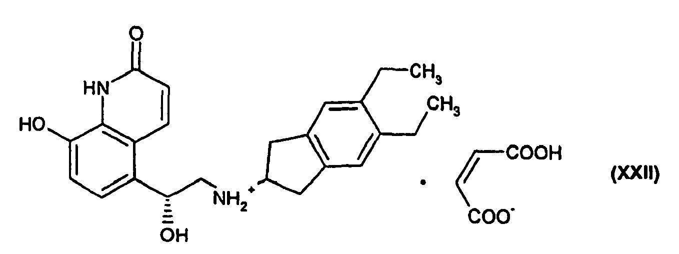

The solvent used in Step (vii) is preferably selected from an alkyl acetate, e.g., C₁-C₆-alkyl acetates, such as ethyl acetate, isopropyl acetate and butyl acetate; alcohols, e.g. C₁-C₆-alkyl alcohols, such as methanol, ethanol, propanol, isopropanol, butanol and pentanol; dimethylformamide; dimethylacetamide; aromatic hydrocarbons, such as toluene and benzene; dialkyl ketones, e.g. acetone and methyl isobutyl ketone; acetonitrile; heterocycles, such as tetrahydrofuran; dialkyl ethers, e.g., diisopropyl ether, 2-methoxyethyl ether and diethylene ether; an acid such as acetic acid and propionic acid; aqueous solvents, such as water; ionic liquids; and chlorinated solvents, such as methylenechloride. A combination of solvents may also be used. More preferably, the solvent is ethanol.

The temperature used in Step (vii) is preferably from about 0 °C to about 70 °C. More preferably, the temperature is from about 10 °C to about 60 °C; and most preferably from about 20 °C to about 50 °C.

As mentioned above, 5-[(R)-2-(5,6-diethyl-indan-2-ylamino)-1-hydroxy-ethyl]-8-hydroxy-(*1H*)-quinolinone-2-one salts can be prepared from 8-hydroxy-(*1H*)-quinolin-2-one or 8-acetoxy-(*1H*)-quinolin-2-one. The third aspect of the present invention involves the preparation of 5-(α-haloacetyl)-8-substituted oxy-(*1H*)-quinolin-2-ones (steps a through c), their reaction with a reducing agent in the presence of a chiral agent to form 8-substituted oxy-5-((*R*)-2-halo-1-hydroxy-ethyl)-(*1H*)-quinolin-2-ones (step d), and their subsequent conversion to 5-[(R)-2-(5,6-diethyl-indan-2-ylamino)-1-hydroxy-ethyl]-8-hydroxy-(*1H*)-quinolinone-2-one salts (steps e through j).

In step (a) 8-hydroxy-(*1H*)-quinolin-2-one or 8-acetoxy-(*1H*)-quinolin-2-one is converted to form 5-acetyl-8-hydroxy-(*1H*)-quinolin-2-one. There are three process variants for this step, namely, step (a)(i), step (a)(ii) and step (a)(iii).

In step (a) (i) 8-hydroxy-(*1H*)-quinolin-2-one is reacted with an acylating agent and a Lewis acid to form 5-acetyl-8-hydroxy-(*1H*)-quinolin-2-one.

In step (a) (ii) 8-hydroxy-(*1H*)-quinolin-2-one is reacted with an acylating agent to form 8-acetoxy-(*1H*)-quinolin-2-one, which is then treated *in situ* with a Lewis acid to form 5-acetyl-8-hydroxy-(*1H*)-quinolin-2-one.

In step (a)(iii) 8-acetoxy-(*1H*)-quinolin-2-one is reacted with a Lewis acid to form 5-acetyl-8-hydroxy-(*1H*)-quinolin-2-one.

The 8-hydroxy-(*1H*)-quinolin-2-one has formula XXIII

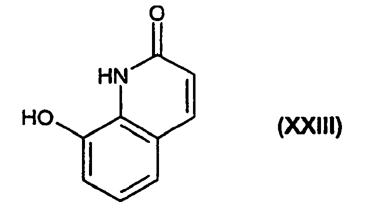

The 5-aceryl-8-hydroxy-(*1H*)-quinolin-2-one has formula XXIV

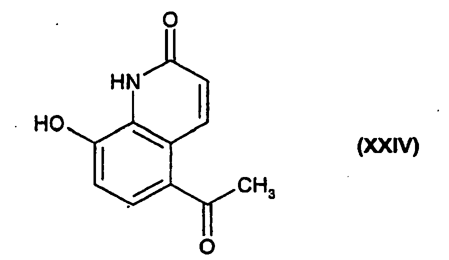

In step (a) the acylating agent, when used, is preferably acetic anhydride or acetyl chloride. The acylating agent is preferably present in an amount of from about 1 molar equivalents to about 1.5 molar equivalents, more preferably about 1.05 molar equivalents, based on the molar equivalents of 8-hydroxy-(*1H*)-quinolin-2-one.

The Lewis acid is preferably selected from boron trifluoride (BF₃), aluminium chloride (AlCl₃), and titanium tetrachloride (TiCl₄)- More preferably, the Lewis acid is aluminium chloride. A combination of Lewis acids may also be used.

The Lewis acid is present in an amount of greater than 2 molar equivalents, based on the molar equivalents of 8-hydroxy-(*1H*)-quinolin-2-one or molar equivalents of 8-acetoxy-(*1H-*quinolin-2-one. Preferably, the Lewis acid is present in an amount of about 3 molar equivalents to about 5 molar equivalents, more preferably from about 3.2 molar equivalents to about 4 molar equivalents.

In one embodiment of the invention, Step (a) is conducted in the presence of a solvent. In another embodiment of the invention, Step (a) is conducted in the absence of a solvent and in the presence of an ionic compound. The ionic compound is an ionic liquid or an alkaline halide.

Preferably a solvent is used in Step (a). The solvent is preferably a solvent compatible with Friedel-Craft conditions. Such solvents are well-known to those skilled in the art and include chlorobenzene, o-dichlorobenzene, 1,2-ethylene dichloride, aliphatic C₆-C₁₂hydrocarbons, e.g., isooctane, heptane and combinations thereof. A combination of solvents may also be used. A preferred solvent for use in Step (a) is o-dichlorobenzene.

Step (a) may be conducted in the absence of a solvent and in the presence of an ionic compound selected from an alkaline halide and an ionic liquid. The alkaline halide is preferably selected from sodium chloride, sodium bromide, lithium chloride and lithium bromide. More preferably, the alkaline halide is sodium chloride. A combination of alkaline halides may also be used.

Ionic liquids are characterized by a positively-charged cation and a negatively-charged anion. Generally, any molten salt or mixture of molten salts is considered an ionic liquid. Ionic liquids typically have essentially no vapour pressure, good heat transfer characteristics, are stable over a wide temperature range and are capable of dissolving a wide range of material in high concentrations. As used herein, "essentially no vapour pressure" means that the ionic liquid exhibits a vapour pressure of less than about 1 mm/Hg at 25 °C, preferably less than about 0.1 mm/Hg at 25 °C.

With respect to the type of ionic liquid, a wide variety of possibilities exist. However, the preferred ionic liquids are liquid at relatively low temperatures. Preferably, the ionic liquid has a melting point of less than 250 °C, more preferably less than 100 °C. Most preferably, the ionic liquid has a melting point of less than 30 °C and is a liquid at room temperature. Preferably, the ionic liquid has a viscosity of less than 500 centipoise (cP), more preferably, less than 300 cP, and most preferably less than 100 cP, as determined at 25 °C.

The cation present in the ionic liquid can be a single species or a plurality of different species. Both of these embodiments are intended to be embraced, unless otherwise specified, by the use of the singular expression "cation". The cations of the ionic liquid include organic and inorganic cations. Examples of cations include quaternary nitrogen-containing cations, phosphonium cations and sulfonium cations.

The quaternary nitrogen-containing cations are not particularly limited and embrace cyclic, aliphatic and aromatic quaternary nitrogen-containing cations. Preferably, the quaternary nitrogen-containing cation is an n-alkyl pyridinium, a dialkyl imidazolium or an alkylammonium of the formula R'₄₋ₓ NHx, wherein x is 0-3 and each R' is independently an alkyl group having 1-18 carbon atoms. It is believed that unsymmetrical cations can provide for lower melting temperatures. The phosphonium cations are not particularly limited and embrace cyclic, aliphatic and aromatic phosphonium cations. Preferably, the phosphonium cations include those of the formula R"₄₋ₓ PHₓ, wherein x is 0-3, and each R" is an alkyl or aryl group, such as an alkyl group having 1-18 carbon atoms or a phenyl group. The sulfonium cations are not particularly limited and embrace cyclic, aliphatic and aromatic sulfonium cations. Preferably, the sulfonium cations include those of the formula R'''₃₋ₓ SHx, wherein x is 0-2 and each R''' is an alkyl or aryl group, such as an alkyl group having 1-18 carbon atoms or a phenyl group. Preferred cations include 1-hexylpyridinium, ammonium, imidazolium, 1-ethyl-3-methylimidazolium, 1-butyl-3-methylimidazolium, phosphonium and *N*-butylpyridinium.

The anion used in the ionic liquid is not particularly limited and includes organic and inorganic anions. Generally the anion is derived from an acid, especially a Lewis acid. The anions are typically metal halides as described in more detail below, boron or phosphorus fluorides, alkylsulfonates including fluorinated alkyl sulfonates, such as nonafluorobutane-sulfonate; and carboxylic acid anions, such as trifluoroacetate and heptafluorobutanoate. The anion is preferably Cl⁻, Br, NO₂⁻, NO₃⁻, AlCl₄⁻, BF₄⁻, PF₆⁻, CF₃COO⁻, CF₃SO₃⁻, (CF₃SO₂)₂N⁻, OAc⁻, CuCl₃⁻ ; GaBr₄⁻, GaCl₄⁻ and SbF₆⁻.

Examples of ionic liquids include, but are not limited to, imidazolium salts, pyridium salts, ammonium salts, phosphonium salts and sulphonium salts. Preferred imidazolium salts have formula XXV 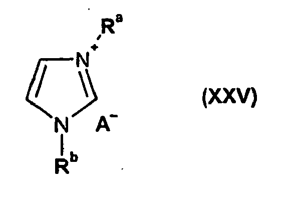 wherein R^{a} and R^{b} are, independently, selected from the group consisting of a C₁-C₁₈-aliphatic group and a C₄-C₁₈-aromatic group; and A- is an anion.

Preferred ammonium salts have formula XXVI 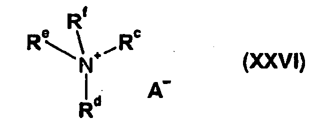 wherein R^{c}, R^{d}, R^{e} and R^{f} are, independently, selected from the group consisting of a C₁-C₁₈-aliphatic group and a C₄-C₁₈-aromatic group; and A- is an anion. Preferably, R^{c}, R^{d}, R^{e} and R^{f} are, independently, selected from the group consisting of ethyl, propyl and butyl.

Preferred phosphonium salts have formula XXVII 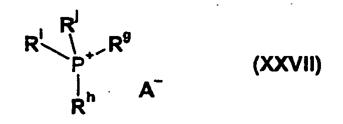 wherein R^{g}, R^{h}, Rⁱ and R^{j} are, independently, selected from the group consisting of a C₁-C₁₈-aliphatic group and a C₄-C₁₈-aromatic group; and A- is an anion. Preferably, R^{g}, R^{h}, Rⁱ and R^{j} are, independently, selected from the group consisting of ethyl and butyl.

Preferred pyridinium salts have formula XXVIII 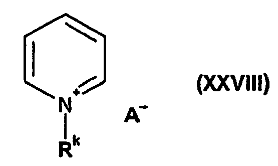 wherein R^{k} is selected from the group consisting of a C₁-C₁₈-aliphatic group and a C₄-C₁₈-aromatic group; and A- is an anion. Preferably R^{k} is ethyl or butyl.

Specific examples of ionic liquids include, but are not limited to, 1-butyl-3-methylimidazolium hexafluorophosphate, 1-hexyl-3-methylimidazolium hexafluorophosphate, 1-octy-3-methylimidazolium hexafluorophosphate, 1-decyl-3-methylimidazolium hexafluorophosphate, 1-dodecyl-3-methylimidazolium hexafluorophosphate, 1-ethyl-3-methylimidazolium *bis*((trifluoromethyl)sulphonyl)-imidate, 1-hexyl-3-methylimidazolium *bis*((trifluoromethyl)sulphonyl)amide, 1-hexylpyridinium tetrafluoroborate, 1-octylpyridinium tetrafluoroborate, 1-butyl-3-methylimidazolium tetrafluoroborate, 1-methy-3-ethyl imidazolium chloride, 1-ethyl-3-butyl imidazolium chloride, 1-methy-3-butyl imidazolium chloride, 1-methy-3-butyl imidazolium bromide, 1-methy-3-propyl imidazolium chloride, 1-methy-3-hexyl imidazolium chloride, 1-methy-3-octyl imidazolium chloride, 1-methy-3-decyl imidazolium chloride, 1-methy-3-dodecyl imidazolium chloride, 1-methy-3-hexadecyl imidazolium chloride, 1-methy-3-octadecyl imidazolium chloride, 1-methy-3-octadecyl imidazolium chloride, ethyl pyridinium bromide, ethyl pyridinium chloride, ethylene pyridinium dibromide, ethylene pyridinium dichloride, butyl pyridinium chloride and benzyl pyridinium bromide.

Preferred ionic liquids are 1-ethyl-3-methyl-imidazolium trifluoroacetate, 1-butyl-3-methylimidazolium trifluoroacetate, 1-ethyl-3-methyl-imidazolium trifluoroacetate, 1-butyl-3-methyl-imidazolium hexafluorophosphate, 1-octyl-3-methyl-imidazolium hexafluorophosphate, 1-hexyl-3-methy-imidazolium hexafluorophosphate, 1-butyl-3-methylimidazolium hexafluorophosphate, 1-butyl-3-methyl-imidazolium tetrafluoroborate, 1-ethyl-3-methyl-imidazolium tetrafluoroborate, 1-octyl-3-methyl-imidazolium bromide, 1-ethyl-3-methyl-imadazolium trifluorosulfonate, 1-butyl-3-methyl-imidazolium trifluorosulfonate,1-butyl-3-methyl-imidazolium trifluoromethanesulfonate, 1-ethyl-3-methyl-imidazolium trifluoromethanesulfonate and 1-ethyl-3-methyl-imidazolium *bis*-(trifluoromethanesulfonyl)-imidate. Most preferably, the ionic liquid is selected from 1-ethyl-3-methyl-imidazolium trifluorosulfonate, 1-butyl-3-methylimidazolium chloride, 1-octyl-3-methyl-imidazolium hexafluorophosphate and 1-hexyl-3-methyl-imidazolium hexafluorophosphate. A combination of ionic liquids may also be used.

Mixtures of ionic compounds and Lewis acids may form reactive liquids at low temperature (see Wasserscheid et al., Angew. Chem. Int. Ed., Vol. 39, pp. 3772-3789 (2000)).

Preferably, the weight ratio of Lewis acid to ionic compound is from about 10 to about 0.1, respectively. More preferably, the ratio of Lewis acid to ionic compound is from about 3 to about 1, respectively.

The temperature used in Step (a) is preferably from about 0 °C to about 160 °C. More preferably, the temperature is from about 10 °C to about 120 °C; and most preferably from about 15 °C to about 110 °C.

The 5-acetyl-8-hydroxy-(1*H*)-quinolin-2-one product prepared in Step (a) may also be present with 7-acetyl-8-hydroxy-(1*H*)-quinolin-2-one having formula XXIX

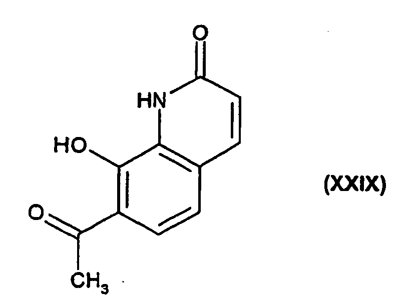

7-Acetyl-8-hydroxy-(*1H*)-quinolin-2-one is surprisingly much more soluble than 5-acetyl-8-hydroxy-(*1H*)-quinolin-2-one. The 5-acetyl-8-hydroxy-(*1H*)-quinolin-2-one may be recovered from the reaction mixture and purified by any of the various techniques known to the art, such as by crystallization or forming a slurry in a solvent. A preferred solvent for forming a slurry is acetic acid.

In the second step, Step (b), the 5-acetyl-8-hydroxy-(*1H*)-quinolin-2-one that is prepared in Step (a) is reacted with a compound having the Formula R-Q in the presence of a base and a solvent to form 5-acetyl-8-substituted oxy-(*1H*)-quinolin-2-one, wherein R is a protecting group and Q is a leaving group.

The 5-acetyl-8-substituted oxy-(*1H*)-quinolin-2-one has formula XXX 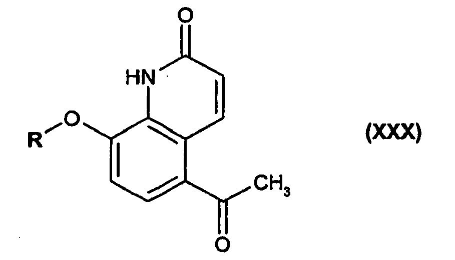 wherein R is a protecting group.

Where reference is made herein to protected functional groups or to protecting groups, the protecting groups may be chosen in accordance with the nature of the functional group, for example as described in Protective Groups in Organic Synthesis, T.W. Greene and P.G.M. Wuts, John Wiley & Sons Inc, Third Edition, 1999, which reference also describes procedures suitable for replacement of the protecting groups by hydrogen.

Preferred protecting groups are phenol protecting groups which are known to those skilled in the art. More preferably, the protecting group is selected from alkyl, alkenyl, aryl, (cycloalkyl)alkyl, arylalkyl; cycloalkyl and a substituted silyl group. The alkyl or aryl group has from 1-24 carbon atoms, more preferably 6-12 carbon atoms. The substituted silyl group is preferably substituted with at least one alkyl group. Most preferably, the protecting group is benzyl or *t*-butyldimethylsilyl.

Preferably, the compound having the formula R-Q is an alkyl halide or substituted alkyl halide, such as α-methylbenzyl bromide, methyl chloride, benzylchloride and benzylbromide. Preferred bases include sodium ethoxide, sodium hydroxide, potassium hydroxide, potassium phosphate, potassium carbonate, potassium hydrogencarbonate, caesium carbonate, pyridine and trialkylamines such as triethylamine, tributhylamine and N,N-diisopropylethylamine. A combination of bases may also be used. Preferred bases are potassium hydroxide, potassium carbonate and potassium hydrogencarbonate. Most preferably, the base is N,N-diisopropylethylamine.

The solvent in Step (b) is preferably selected from an alkyl acetate, e.g., C₁-C₆-alkyl acetates, such as ethyl acetate, isopropyl acetate and butyl acetate; lower alkyl alcohols, e.g., C₁-C₆-alkyl alcohols, such as methanol, ethanol, propanol, butanol and pentanol; dimethylformamide; dimethylacetamide; dialkyl ketones, e.g., acetone and methyl isobutyl ketone; acetonitrile; heterocycles, such as tetrahydrofuran; dialkyl ethers, e.g., diisopropyl ether, 2-methoxyethyl ether and diethylene ether; aqueous solvents, such as water; ionic liquids; and chlorinated solvents, such as methylenechloride. A combination of solvents may also be used.

A preferred solvent for use in Step (b) is an acetone/water mixture. A preferred volume ratio of acetone to water is from 10:90 to 90:10, respectively. More preferably, the volume ratio of acetone to water is from 20:80 to 80:20, respectively. Most preferably, the volume ratio of acetone to water is about 75:25.

The temperature used in Step (b) is preferably from about 20°C to about 90 °C. More preferably, the temperature is from about 30 °C to about 80 °C; and most preferably from about 50°C to about 70 °C.

The 5-acetyl-8-substituted oxy-(*1H*)-quinolin-2-one is preferably 5-acetyl-8-benzyloxy-(*1H*)-quinolin-2-one.

Optionally, the 5-acetyl-8-substituted oxy-(*1H*)-quinolin-2-one product may be purified by any of the various techniques known to the art, such as by crystallization.

In the third step, Step (c), the 5-acetyl-8-substituted oxy-(*1H*)-quinolin-2-one that is prepared in Step (b) is reacted with a halogenating agent in the presence of a solvent to form 5-(α-haloacetyl)-8-substituted oxy-(*1H*)-quinolin-2-one.

The 5-(α-haloacetyl)-8-substituted oxy-(*1H*)-quinolin-2-one has formula X as hereinbefore defined wherein R is a protecting group; and X is a halogen.

The halogenating agent may be any compound or combination of compounds that provide a halogen atom *in situ.* Preferred halogenating agents include sodium bromate and hydrobromic acid, bromine, *N*-bromosuccinimide, *N*-chlorosuccinimide, iodine, chlorine, sulfuryl chloride, benzyltrimethylammoniumdichloroiodate, copper chloride, pyridinium tribromide, tetraalkylammonium tribromide, iodine chloride, hydrochloric acid and an oxidating agent, such as oxone, hydrogen peroxide and monoperoxyphthalic acid. A combination of halogenating agents may also be used. Most preferably, the halogenating agent is benzyltrimethylammoniumdichloroiodate. It is within the scope of the invention to use sulfuryl chloride with methanol.

The solvent used in Step (c) is preferably selected from an acid, e.g., carboxylic acids, such as acetic acid, trifluoroacetic acid and propionic acid; an alkyl acetate, e.g., C₁-C₆-alkyl acetates, such as ethyl acetate, isopropyl acetate and butyl acetate; dimethylformamide; dimethylacetamide; aromatic hydrocarbons, such as toluene and benzene; acetonitrile; heterocycles, such as tetrahydro-furan; dialkyl ethers, e.g., diisopropyl ether, 2-methoxyethyl ether and diethylene ether; ionic liquids; and chlorinated solvents, such as methylenechloride. A combination of solvents may also be used. A preferred solvent for use in Step (c) is acetic acid.

The temperature used in Step (c) is preferably from about 10 °C to about 160 °C. More preferably, the temperature is from about 20 °C to about 120 °C; and most preferably from about 60 °C to about 75 °C.

The 5-(α-haloacetyl)-8-substituted oxy-(*1H*)-quinolin-2-one product is preferably 5-(α-chloroacetyl)-8-benzyloxy-(*1H*)-quinolin-2-one.

Optionally, the 5-(α-haloacetyl)-8-substituted oxy-(*1H*)-quinolin-2-one product may be purified by any of the various techniques known to the art, such as by crystallization.

In the second aspect the present invention, which provides a process for preparing 5-[(R)-2-. (5,6-diethyl-indan-2-ylamino)-1-hydroxy-ethyl]-8-hydroxy-(*1H*)-quinolinone-2-one salts, the 8-substituted oxy-5-((*R*)-2-halo-1-hydroxy-ethyl)-(*1H*)-quinolin-2-one is converted to the 8-substituted oxy-5-(*R*)-oxiranyl-(*1H*)-quinolin-2-one using steps (d) and (e). These steps correspond to steps (i) and (ii) of the process for 8-substituted oxy-5-(*R*)-oxiranyl-(*1H*)-quinolin-2-ones or acceptable solvates thereof that has been described in detail above.

Step (d) is carried out in accordance with the description of the first aspect of the process of the present invention. It also corresponds to step (i) of the second aspect of the process of the present invention.

Steps (e) through (j) are carried out in accordance with the description of steps (ii) through (vii) of the second aspect of the present invention.

The following non-limiting examples illustrate further aspects of the invention.

### EXAMPLES

### Example 1

### Preparation of 5-acetyl-8-hydroxy-(1H)-quinolin-2-one

Aluminium chloride (93.3 g, 700 mmol, 3.5 eq.) is suspended in 1,2-dichlorobenzene (320 mL). The suspension is maintained at 20-25 °C and 8-hydroxy-(*1H*)-quinolin-2-one (32.24 g, 200 mmol, 1.0 eq.) is added in 5 portions (40 minutes, IT max. 25 °C). Acetic anhydride (21.4 g, 210 mmol, 1.05 eq.) is slowly added (30 minutes, IT max. 20 °C) and the addition funnel is rinsed with a small amount of 1,2-dichlorobenzene. The suspension is stirred for 30 minutes at 20-25 °C. HPLC control reveals complete conversion to 8-acetoxy-(*1H*)-quinolin-2-one. The mixture is heated to IT = 80 °C while purging the head-space with a stream of nitrogen. HCl evolution is noticed upon reaching IT = 40 °C. The reaction mixture is stirred for 1 hour at IT = 80 °C. HPLC control reveals almost complete conversion to 5-acetyl-8-hydroxy-(*1H*)-quinolin-2-one (3.1% O-acetyl intermediate, 10.8% ortho-isomer). The reaction mixture is poured hot (80 °C) over water (800 mL). Water (100 ml) is added in the reaction vessel and brought to reflux temperature. After 15 minutes at reflux temperature, the suspension is added to the previous quench suspension. The mixture is maintained for 15 minutes at IT = 80 °C and then hot filtered. The yellow product is rinsed with water (2 x 200 mL, 50 °C), rinsed with acetone (50 mL) and then dried overnight under vacuum at 70 °C. Yield: 33.32 g (82.0%). Purity: 95-97%.

### Example 2

### Preparation and purification of 5-acetyl-8-hydroxy-(1H)-quinolin-2-one

8-Hydroxy-(*1H*)-quinolin-2-one (32.24 g, 200 mmol, 1.0 eq) is suspended in 1,2-dichlorobenzene (300 mL). The suspension is maintained at 20-25 °C and aluminium chloride (93.3 g, 700 mmol, 3.5 eq.) is added in portions (30 minutes, IT max. 25 °C). Acetic anhydride (21.4 g, 210 mmol, 1.05 eq.) is slowly added (30 minutes, IT max. 20 °C) and the addition funnel is rinsed with a small amount of 1,2-dichlorobenzene. The suspension is stirred for 30 minutes at 20-25 °C. HPLC control reveals complete conversion to 8-acetoxy-(*1H*)-quinolin-2-one. The mixture is heated to IT = 80 °C while purging the head-space with a stream of nitrogen. HCl evolution is noticed upon reaching IT = 40 °C. The reaction mixture is stirred for 1 hour at IT = 80 °C. HPLC control reveals almost complete conversion to 5-acetyl-8-hydroxy-(*1H*)-quinolin-2-one (1.8% O-acetyl intermediate, 7.2% ortho-isomer). The reaction mixture is heated to IT = 90°C and poured hot (90 °C) over water (645 mL). Water (100 mL) is added in the reaction vessel and brought to reflux temperature. After 15 minutes at reflux temperature, the suspension is added to the previous quench suspension. The mixture is maintained for 15 minutes at IT = 80 °C and is hot filtered. The yellow product is rinsed with water (2 x 200 mL, 50 °C). The crude product (70.1 g) is suspended in acetic acid (495 mL) and the suspension is heated to reflux temperature for 30 minutes. The suspension is cooled down to IT = 20°C and then filtered. The product is washed with acetic acid/water 1/1 (60 mL) and washed with water (5 x 100 mL) before being dried at 70°C under vacuum to yield the title compound in 75% yield (31.48 g) and with 99.9% purity.

### Example 3

### Preparation of 5-acetyl-8-hvdroxy-(1H)-quinolin-2-one

5-Acetyl-8-hydroxy-(*1H*)-quinolin-2-one is prepared according to the procedure set forth in Example 1 except that 3 eq. of aluminium chloride is used instead of 3.5 eq. of aluminium chloride. The yield of the title compound is approximately 84%.

### Example 4

### Preparation of 5-acetyl-8-hydroxy-(1H)-quinolin-2-one from 8-acetoxy-(1H)-quinolin-2-one

8-Acetoxy-(*1H*)-quinolin-2-one (6.1 g, 30 mmol, 1.0 eq.) is suspended in 1,2-dichlorobenzene (80 mL). The suspension is warmed to 80 °C and aluminium chloride (12.0 g, 90 mmol, 3.0 eq.) is added in portions. The reaction is stirred for 1 hour at IT = 80 °C. HPLC control reveals almost complete conversion to 5-acetyl-8-hydroxy-(*1H*)-quinolin-2-one. The reaction mixture is poured hot (80 °C) over water (100 mL). Water (30 mL) is added in the reaction vessel and then brought to reflux temperature. After 15 minutes at reflux temperature, the suspension is added to the previous quench suspension. The mixture is maintained for 15 minutes at IT = 80°C and then hot filtered. The yellow product is rinsed with water (2 x 50 mL, 50 °C) and then dried overnight under vacuum at 80 °C. Yield: 4.32 g (79.0%). Purity: 95%.

### Example 5

### Preparation of 5-acetyl-8-benzyloxy-(1H)-quinolin-2-one

[Crude 5-acetyl-8-hydroxy-(*1H*)-quinolin-2-one (8.13 g, 40 mmol, 1.0 eq.) is added to N-N,diisopropylethylamine (6.46 g, 50 mmol, 1.25 eq.) and acetone (64 mL). The suspension is heated to reflux temperature and water is added (8.2 mL). Benzylbromide (7.52 g, 44 mmol, 1.10 eq.) is added drop-wise and the reaction is maintained for 6-7 hours at reflux temperature until all starting material has reacted. Water (20 mL) is added at IT = 58 °C and the mixture is cooled down to 20-25 °C. The product is filtered, washed with acetone/water (1/1, 2 x 8.5 mL) and then with water (4 x 8 mL). The crude product is dried overnight under vacuum (60 °C). Yield: 10.77 g (91.7%). Purity of the crude product: 99.5%. The product may be recrystallised from acetone/water.

### Example 6

### Preparation of 5-(α-chloroacetyl)-8-(phenylmethoxyl)- (1H)-quinolin-2-one

A 3 L, 4-necked flask equipped with a mechanical stirrer, thermometer, addition funnel and refluxing condenser is charged with 40 g 8-(phenylmethoxy)-5-acetyl-(*1H*)-quinolin-2-one and 400 mL acetic acid under an atmosphere of nitrogen. To this yellow solution is added 94.93 g benzyl-trimethylammoniumdichloroiodate and 200 mL acetic acid. The resulting suspension is heated under stirring to an internal temperature of 65-70 °C. The mixture is stirred at this temperature until an in-process control shows complete conversion to 5-chloroacetyl-8-phenylmethoxy-(*1H*)-quinolin-2-one. The mixture is then cooled to a temperature of 40-45 °C. Within 30-60 minutes, 400 mL water is added. The resulting suspension is stirred at 20-25°C for 30-60 minutes and then 300 g of a 5%(w/w) solution of NaHSO₃ in water is added within 30 to 60 minutes at a temperature of 15 to 20 °C. At the end of the addition a test for the presence of I₂ is negative. Crude 5-(α-chloroacecyl)-8-(phenylmethoxy)- (*1H*)-quinolin-2-one is isolated by filtration and purified by crystallisation from acetic acid. Drying in a vacuum oven at 50°C gives 39.3 g of pure 5-(α-chloroacetyl)-8-(phenylmethoxy)- (*1H*)-quinolin-2-one.

### Example 7

### Preparation of 8-(phenylmethoxy)-5-((R)-2-chloro-1-hydroxy-ethyl)-(1H)-quinolin 2-one

In a 3-necked flask 11 mg (1S,2S)-(+)-N-p-tosyl-1,2-diphenylethylendiamine and 9 mg (RuCl₂(p-cymene)]₂ are dissolved in10 ml of methanol/dimethylformamide (95/5 v/v). To the resulting orange solution 9µl triethylamine are added and the mixture is heated to reflux for 1 hour 30 minutes. After cooling to 30°C, 1 g 8-Benzyloxy-5-(2-chloroacetyl)-*1H*-quinolin-2-one followed by 10 ml of methanol/dimethylformamide (95/5 v/v) are added. A mixture of 0.69 ml formic acid and 5.1 ml triethylamine is added and the resulting suspension is stirred until an in process control shows complete conversion to 8-(phenyl-methoxy)-5-((*R*)-2-chloro-1-hydroxy-ethyl)-(*1H*)-quinolin-2-one. The reaction mixture is then concentrated in a rotary evaporator, the residue dissolved in 2.5 ml tetrahydrofuran : methanol 9:1 and the product is isolated by addition of 7.2 ml HCl 0.5 N. Drying over night in a vacuum drier gives 993 mg of 8-(phenylmethoxy)-5-((*R*)-2-chloro-1-hydroxy-ethyl)-(*1H*)-quinolin-2-one.

Alternatively, in a 3-necked flask are placed 5 g of 8-(phenylmethoxy)-5-chloroacetyl-(*1H*)-quinolin-2-one, 97 mg of RuCl[(1*S*,2*S*)*-p-*TsN-CH(C₆H₅)CH(C₆H₅)-NH₂](η⁶*-p-*cymene) and 100 mL of a mixture methanol: dimethylformamide 95:5 under an atmosphere of nitrogen. A pre-formed mixture of 4.21 g formic acid and 18.52 g triethylamine is added at 30 - 34 °C under agitation. The reaction mixture is stirred at an internal temperature of 30 °C until an in process control shows complete conversion to 8-(phenylmethoxy)-*S*-((*R*)-2-chloro-1-hydroxy-ethyl)-(*1H*)-quinolin-2-one. Then the reaction mixture is concentrated in a rotary evaporator, the residue dissolved in 25ml tetrahydrofuran : methanol 9:1 and the product is isolated by addition of 72 ml HCl 0.5 N. Drying over night in a vacuum drier gives 4.76 g of 8-(phenyl-methoxy)-5-((*R*)-2-chloro-1-hydroxy-ethyl)-(*1H*)-quinolin-2-one.

As a further alternative, in a 3-necked flask are placed 40 g of 8-(phenylmethoxy)-5-chloroacetyl-(*1H*)-quinolin-2-one, 776 mg of RuCl[(1*S*,2*S*)*-p-*TsN-CH(C₆H₅)CH(C₆H₅)-NH₂](η⁶*-p-*cymene) and 800 ml of a mixture methanol : dimethylformamide 9:1 under an atmosphere of nitrogen. A pre-formed mixture of 9.2 ml formic acid and 68 ml triethylamine is added at 10 - 30 °C under agitation. The reaction mixture is stirred at an internal temperature of 30 °C until an in process control shows complete conversion to 8-(phenylmethoxy)-5-((*R*)-2-chloro-l-hydroxy-ethyl)-(*1H*)-quinolin-2-one. In order to consume any remaining formic acid, 180 ml acetone are added and the internal temp. is raised to 40 °C. The mixture is stirred at 40 °C until an in process control shows <0.01%(w/w) formic acid. Then 31.4ml Acetic acid are added and the reaction mixture is concentrated in a rotary evaporator to a volume of 300ml, the residue dissolved in 250ml tetrahydrofuran and the product is isolated by addition of 720ml water. Drying over night in a vacuum drier gives 37g of 8-(phenyl-methoxy)-5-((R)-2-chloro-1-hydroxy-ethyl)-(1*H*)-quinolin-2-one.

As a yet further alternative, in a 3-necked flask are placed 10 g of 8-(phenylmethoxy)-5-chloroacetyl-(*1H*)-quinolin-2-one, 194.2 mg of RuCl[(1*S*,2*S*)-*p-*TsN-CH(C₆H₅)CH(C₆H₅)-NH_{2]}(η⁶-*p-*cymene) and 200 ml of a mixture methanol : dimethylacetamide 9:1 under an atmosphere of nitrogen. A preformed mixture of 2.3 ml formic acid and 17 ml triethylamine is added at 10 - 30 °C under agitation. The reaction mixture is stirred at an internal temperature of 30 °C until an in process control shows complete conversion to 8-(phenylmethoxy)-5-((*R*)-2-chloro-1-hydroxy-ethyl)-(*1H*)-quinolin-2-one. Then 45 ml acetone are added and the internal temperature is raised to 40 °C. The mixture is stirred at 40 °C until an in process control shows <0.01%(w/w) formic acid. Then 7.9 ml acetic acid are added and the reaction mixture is concentrated in a rotary evaporator to a volume of 75 ml, the residue dissolved in 62.5 ml tetrahydrofuran and the product is isolated by adding 150 ml water and filtration. Drying over night in a vacuum drier gives 9.34 g of 8-(phenyl-methoxy)-5-((*R*)-2-chloro-1-hydroxy-ethyl)-(1*H*)-quinolin-2-one.

### Example 8

### Preparation of 8-(phenylmethoyy)-5-(R)-oxiranyl-(1H)-quinolin-2-one

A 4-necked flask equipped with a mechanical stirrer, thermometer, addition funnel and refluxing condenser is charged with 15 g 8-(phenylmethoxy)-5-((*R*)-2-chloro-1-hydroxyethyl)-(*1H*)-quinolin-2-one, 15.72 g potassium carbonate, 375 mL 2-butanone and 3.75 mL water. The mixture is heated under stirring to reflux. Refluxing is maintained until an in-process control shows complete conversion of 8-phenylmethoxy-5-((*R*)-2-chloro-1-hydroxyethyl)-(*1H*)-quinolin-2-one to 8-phenylmethoxy-5-(*R*)-oxiranyl-(1*H*)-quinolin-2-one. When the reaction is complete, the hot reaction mixture is filtered to remove the inorganic salts. The residue is washed with several portions of 2-butanone, and the combined mother liquor and 2-butanone washings are concentrated to a volume of about 180 mL. To the resulting suspension is added 210 mL toluene. This suspension is again heated to IT = 70 to 80- °C. 8-(phenylmethoxy)-5-(*R*)-oxiranyl-(*1H*)-quinolin-2-one is isolated by cooling down to 0 °C, filtration and crystallisation of the crude product from toluene. Drying over night at 50°C gives 11 g of 8-(phenylmethoxy)-5-(*R*)-oxiranyl-(*1H*)-quinolin-2-one.

Alternatively, a 4-necked flask equipped with a mechanical stirrer, thermometer, addition funnel and refluxing condenser is charged with 50 g 8-(phenylmethoxy)-5-((*R*)-2-chloro-1-hydroxy-ethyl)-(*1H*)-quinolin-2-one, 52.42 g potassium carbonate, 2.5 L acetone and 25 mL water. The mixture is heated under stirring to reflux. Refluxing is maintained until an in-process control shows complete conversion of 8-phenylmethoxy-5-((*R*)-2-chloro-1-hydroxyethyl)-(*1H*)-quinolin-2-one to 8-phenylmethoxy-5-(*R*)-oxiranyl-(*1H*)-quinolin-2-one. When the reaction is complete, the hot reaction mixture is filtered to remove the inorganic salts. The-residue is washed with several portions of acetone, and the combined mother liquor and acetone washings are concentrated to a volume of about 450 ml. To the resulting suspension are added 235 mL heptanes. This suspension is stirred for 2-3 hours at 0 - 5°C and the crude product is isolated by filtration and crystallised from toluene. Drying over night at 50°C gives 37g of 8-(phenylmethoxy)-5-(*R*)-oxiranyl-(*1H*)-quinolin-2-one.

### Example 9

### Preparation of 5-[(R)-2-(5,6-diethyl-indan-2-ylamino)-1-hardroxy-ethyl]-8-phenylmethoxy-(1H)-quinolin-2-one benzoate

A 1 L, 4-necked flask equipped with a mechanical stirrer, thermometer, addition funnel and refluxing condenser is charged with 30.89 grams of 2-amino-5,6-diethylindan and diethylene glycol dimethyl ether (93 mL). To this solution is added 36.4 grams of 8-phenyl-methoxy-5-(R)-oxiranyl-*1H*-quinolin-2-one. The resulting suspension is heated to a temperature of 110 °C and stirred at this temperature for 15 hours. The resulting brown solution is cooled to 70 °C. At 70 °C, 210 mL of ethanol is added followed by a solution of 30.3 grams of benzoic acid in 140 mL of ethanol. The solution is cooled to 45-50 °C and seeded. The suspension is cooled to 0-5 °C. The crude 8-phenylmethoxy-5-[(R)-2-(5,6-diethyl-indan-2-ylamino)-1-hydroxy-ethyl]-*1H*-quinolin-2-one benzoate is isolated by filtration and washed with 150 mL of ethanol in three portions. The wet filter cake is purified by re-crystallization from 1400 mL of ethanol, which gives 50.08 g pure 8-phenylmethoxy-5-[(R)-2-(5,6-diethyl-indan-2-ylamino)-1-hydroxy-ethyl]-*1H*-quinolin-2-one benzoate as a white crystalline powder.

### Example 10

### Preparation of 5-[(R)-2-(5,6-diethl-indan-2-ylamino)-1-hydroxy-ethyl]-8-hydroxy-1H quinolin-2-one maleate

A 1 L hydrogenation vessel is charged with 40 grams of 8-phenylmethloxy-5-[(R)-2-(5,6-diethyl-indan-2-ylamino)-1-hydroxy-ethyl]-*1H*-quinolin-2-one benzoate and 400 mL of acetic acid. Palladium on charcoal 5% (5.44 g) is added and the reaction mass is hydrogenated for 2-8 hours until complete conversion to 5-[(R)-2-(5,6-diethyl-indan-2-ylamino)-1-hydroxyethyl]-8-hydroxy-*1H*-quinolin-2-one. The mixture is filtered over a pad of filter-aid. The filtrate is concentrated at 50-60 °C under vacuum (100 mbar) to a volume of 70-90 mL. This residue is dissolved in 400 mL of ethanol and heated to 50-60 °C. A solution of 11.6 g maleic acid in 24 mL ethanol is added and the resulting clear solution is seeded at an internal temperature of 50 °C with a suspension of 350 mg micronised 5-[(R)-2-(5,6-diethyl-indan-2-ylamino)-1-hydroxy-ethyl]-8-hydroxy-*1H*-quinolin-2-one maleate in 20 mL isopropanol. The product is crystallized by slow cooling to 0-5 °C. Filtration and washing with 50 mL of ethanol followed by 25 mL of isopropanol provides 65 g crude 5-[(R)-2-(5,6-diethyl-indan-2-ylamino)-1-hydroxy-ethyl]-8-hydroxy-*1H*-quinolin-2-one maleate which is further purified by crystallization from 1.36 L of ethanol. This gives 24.3 g pure 5-[(R)-2-(5,6-diethyl-indan-2-ylamino)-1-hydroxy-ethyl]-8-hydroxy-*1H*-quinolin-2-one maleate as a white crystalline powder.

### Example 11

### Purity and Yield of Different Salts of 5-[(R)-2-(5,6-diethyl-indan-2-ylamiuno)-1-hydroxy-ethyl]-8-substituted oxy-(1H)-quinolin-2-one

A 1 L, 4-necked flask equipped with a mechanical stirrer, thermometer, addition funnel and refluxing condenser is charged with 30.89 grams of 2-amino-5,6-diethylindan and diethylene glycol dimethyl ether. To this solution is added 36.4 grams of 8-phenyl-methoxy-5-(R)-oxi- , ranyl-*1H*-quinolin-2-one. The resulting suspension is heated to a temperature of 110 °C and stirred at this temperature for 15 hours. The resulting brown solution is cooled to 70 °C.

The reaction is conducted as follows: 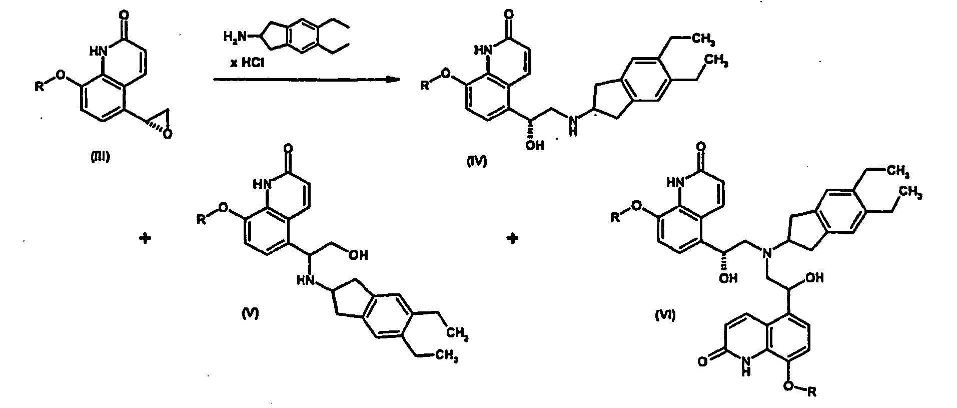 where R is Bn.

As determined by HPLC, the reaction mixture contains 68.7% of a compound having formula IV, 7.8% of a compound having formula V, and 12.4% of a compound having formula VI. The reaction mixture is split in equal portions and each portion is individually treated with an acid selected from benzoic acid, maleic acid, succinic acid, fumaric acid, tartaric acid and hydrochloric acid. The results are summarized in Table 1 as follows:

**TABLE 1**

| Salt | Purity [%(Area)] | Yield [%] |
|---|---|---|
| Benzoate | 96 | 60 |
| Maleate | 98 | 28 |
| Fumarate | 97 | 48 |
| Succinate | 98 | 30 |
| Tartrate | 98 | 25 |
| Hydrochloride | 87 | 25 |

As set forth in Table 1, the percent yield is based on the amount of 8-substituted oxy-5-(R)-oxiranyl-(*1H*)-quinolin-2-one, and the purity is based on the salt having formula IV and is determined by HPLC.

## Claims

1. A process for preparing 8-substituted oxy-5-((*R*)-2-halo-1-hydroxy-ethyl)-(*1H*)-quinolin-2-ones or acceptable solvates thereof comprising reacting a 5-(α-haloacetyl)-8-substituted oxy-(*1H*)-quinolin-2-one with a reducing agent in the presence of a chiral agent and a base to form a 8-(substituted oxy)-5-((*R*)-2-halo-1-hydroxy-ethyl)-(*1H*)-quinolin-2-one, said chiral agent having a formula I or II 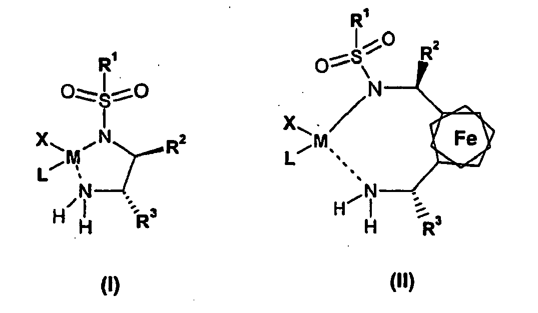 wherein
M is Ru, Rh, Ir, Fe, Co or Ni;
L is C₆-C₂₄-aryl or a C₆-C₂₄-aryl-C₁-C₁₀-aliphatic residue, in either case being optionally linked to a polymer;
X is hydrogen or halo;
R¹ is a C₁-C₁₀-aliphatic, C₃-C₁₀-cycloaliphatic, C₃-C₁₀-cycloaliphatic- C₁C₁₀-aliphatic, C₆-C₂₄-aryl, C₆-C₂₄-aryl- C₁-C₁₀-aliphatic residue or a 4- to 12-membered heterocyclic group, which, in each case, is optionally linked to a polymer; and
R²and R³ are phenyl,
or R²and R³ together with the carbon atom to which they are attached form a cyclohexane or cyclopentane ring.

2. A process according to Claim 1, wherein the chiral agent has formula I or II, wherein
M is ruthenium;
L is isopropylmethylbenzene, benzene, hexamethylbenzene or mesitylene;
X is hydrogen or halo;
R¹ is phenyl, 2- or 3- or 4-pyridyl, 4'-chloro-4-phenoxy-phenyl, 4-phenoxy-phenyl, 5-dimethylamino-1-naphthyl, 5-nitro-1-naphthyl, 2-, 3-, 4-nitrophenyl, 4-vinylphenyl, 4-biphenylyl, 9-anthracenyl, 2-, 3- or 4-hydroxyphenyl, tolyl, phenanthryl, benzo[1,3]-dioxole, dimethyl(naphthalene-1-yl)-amine, mono to tristrifluoromethylphenyl, chrysenyl, perylenyl or pyranyl; and
R² and R³ are both phenyl.

3. A process according to Claim 1 or 2, wherein the chiral agent is a ruthenium based agent and the reducing agent is selected from the group consisting of 2-propanol, 3-pentanol and formic acid.

4. A process according to any preceding Claim, wherein the chiral agent is RuCl[(*1S*,*2S*)-*p-*TsN-CH(C₆H₅)CH(C₆H₅)-NH₂](η⁶-*p*-cymene).

5. A process according to any preceding Claim, wherein the temperature used is from -10 °C to 80 °C, preferably from 0 °C to 50 °C.

6. A process according to any preceding Claim, wherein the 8-substituted oxy-5-((*R*)-2-halo-1-hydroxy-ethyl)-(*1H*)-quinolin-2-one is 8-phenylmethoxy-5-((*R*)-2-chloro-1-hydroxyethyl)-(*1H*)-quinolin-2-one.

7. A process for preparing 5-[(R)-2-(5,6-diethyl-indan-2-ylamino)-1-hydroxy-ethyl]-8-hydroxy-(*1H*)-quinolinone-2-one salts comprising:
(i) reacting a 5-(α-haloacetyl)-8-substituted oxy-(1*H*)-quinolin-2-one with a reducing agent in the presence of a chiral agent and a base to form a 8-substituted oxy-5-((*R*)-2-halo-1-hydroxy-ethyl)-(*1H*)-quinolin-2-one, said chiral agent having a formula I or II as defined in claim 1
(ii) treating the 8-substituted oxy-5-((*R*)-2-halo-1-hydroxy-ethyl)-(*1H*)-quinolin-2-one with a base in the presence of a solvent to form a 8-substituted oxy-5-(*R*)-oxiranyl-(*1H*)-quinolin-2-one of formula III 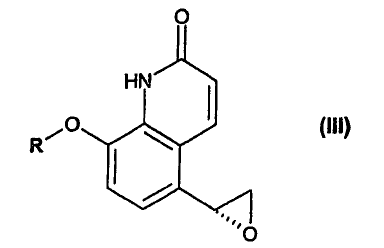 wherein R is a protecting group;
(iii) reacting the 8-substituted oxy-5-(R)-oxiranyl-(*1H*)-quinolin-2-one of formula III where R is as hereinbefore defined, with 2-amino-(5-6-diethyl)-indan to form a reaction mixture containing compounds having formulae IV, V and VI 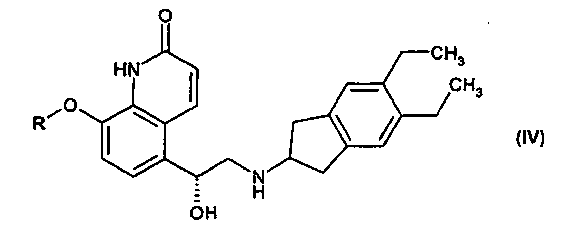 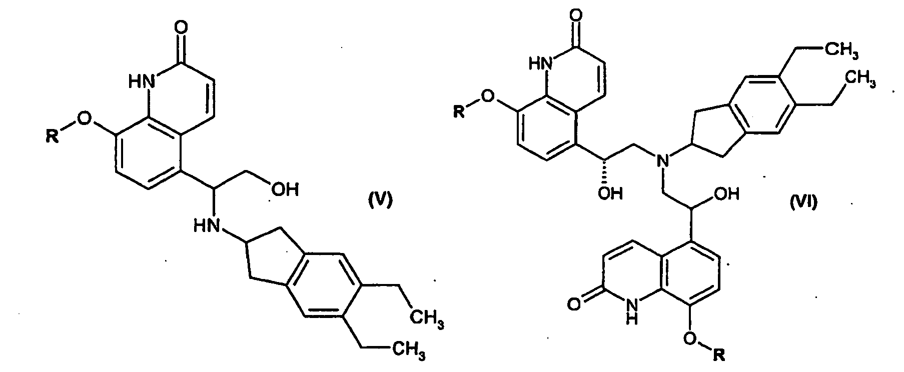 wherein R is a protecting group;
(iv) treating the reaction mixture prepared in Step (iii) with an acid in the presence of a solvent to form a corresponding salt;
(v) isolating and crystallizing a salt having formula VII 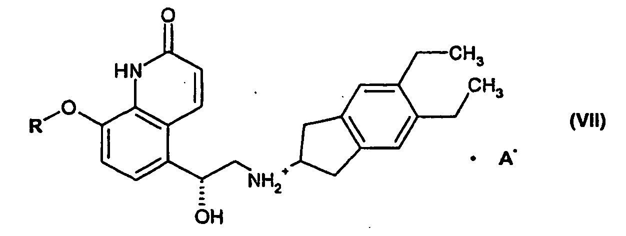 wherein R is a protecting group and A- is an anion;
(vi) removing the protecting group from the salt having formula VII in the presence of a solvent to form a salt having Formula VIII 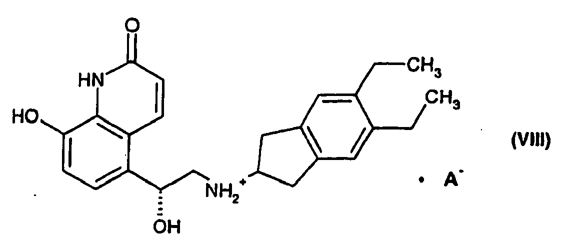 wherein A- is an anion; and
(vii) treating the salt having formula VIII with an acid in the presence of a solvent to form a 5-[(R)-2-(5,6-diethyl-indan-2-ylamino)-1-hydroxy-ethyl]-8-hydroxy-(*1H*)-quinolin-2-one salt having formula IX 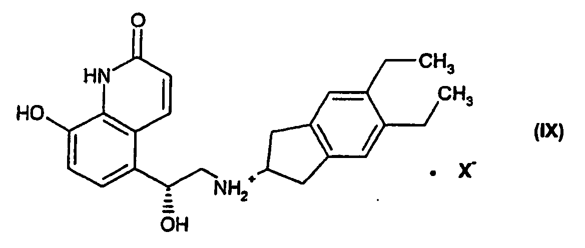 wherein X- is an anion.

8. A process according to Claim 7, wherein the reducing agent is formic acid.

9. A process according to Claim 7 or 8, wherein the base used in step (ii) is ethoxide, sodium hydroxide, potassium phosphate, potassium carbonate, potassium hydrogencarbonate, caesium carbonate or a mixture thereof.

10. A process for preparing 5-[(R)-2-(5,6-diethyl-indan-2-ylamino)-1-hydroxy-ethyl]-8-hydroxy-(*1H*)-quinolin-2-one salts comprising:
(a) reacting
(i) 8-hydroxy-(*1H*)-quinolin-2-one with an acylating agent and a Lewis acid to form 5-aceryl-8-hydroxy-(*1H*)-quinolin-2-one; or
(ii) 8-hydroxy-(*1H*)-quinolin-2-one with an acylating agent to form 8-acetoxy-(*1H*)-quinolin-2-one, and treating, in-situ, the 8-acetoxy-(*1H*)-quinolin-2-one with a Lewis acid to form 5-acetyl-8-hydroxy-(*1H*)-quinolin-2-one; or
(iii) 8-aceroxy-(*1H*)-quinolin-2-one with a Lewis acid to form-5-acetyl-8-hydroxy-(*1H*)-quinolin-2-one;
(b) reacting the 5-aceryl-8-hydroxy-(*1H*)-quinolin-2-one prepared in Step (a) with a compound having the formula R-Q in the presence of a base and a solvent to form 5-acetyl-8-substituted oxy-(*1H*)-quinolin-2-one, wherein R is a protecting group and Q is a leaving group;
(c) reacting the 5-acetyl-8-substituted oxy-(*1H*)-quinolin-2-one with a halogenating agent in the presence of a solvent to form a 5-(α-haloacetyl)-8-substituted oxy-(*1H*)-quinolin-2-one;
(d) reacting the 5-(α-haloacetyl)-8-substituted oxy-(*1H*)-quinolin-2-one with a reducing agent in the presence of a chiral agent and a base to form 8-substituted oxy-5-((*R*)-2-halo-1-hydroxy-ethyl)-(1*H*)-quinolin-2-one, said chiral agent having a formula I or II as defined in claim 1
(e) treating the 8-substituted oxy-5-((*R*)-2-halo-1-hydroxy-ethyl)-(*1H*)-quinolin-2-one with a base in the presence of a solvent to form a 8-substituted oxy-5-(*R*)-oxiranyl-(*1H*)-quinolin-2-one of formula III 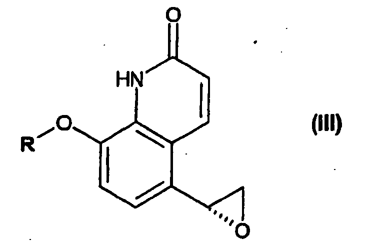 wherein R is a protecting group;
(f) reacting the 8-substituted oxy-5-(R)-oxiranyl-(*1H*)-quinolin-2-one of formula III where R is as hereinbefore defined, with 2-amino-(5-6-diethyl)-indan to form a reaction mixture containing compounds having formulae IV, V and VI 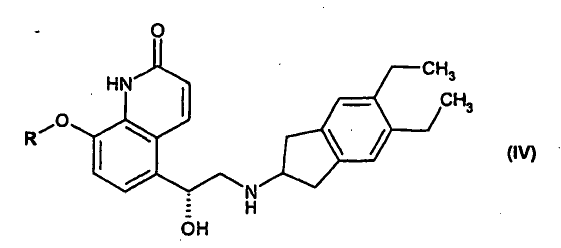 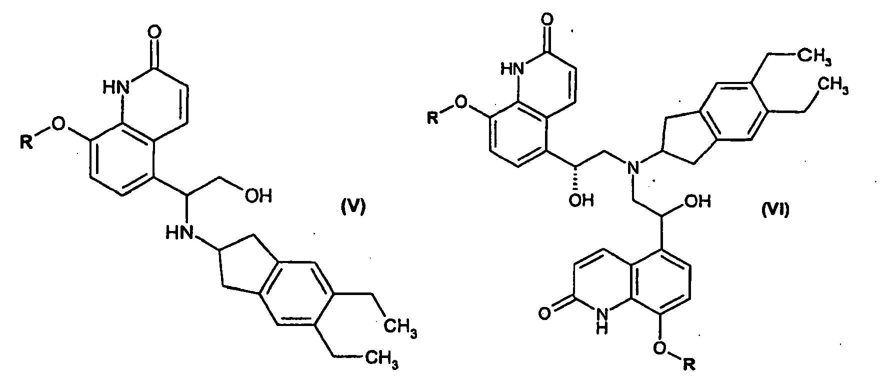 wherein R is a protecting group;
(g) treating the reaction mixture prepared in Step (f) with an acid in the presence of a solvent to form a corresponding salt;
(h) isolating and crystallizing a salt having formula VII 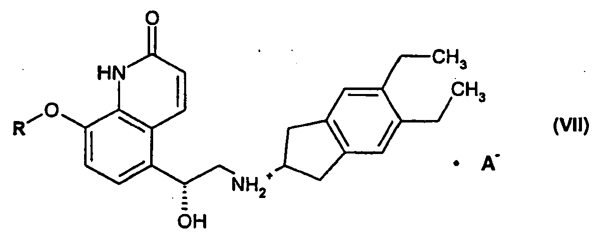 wherein R is a protecting group and A⁻ is an anion;
(i) removing the protecting group from the salt having formula VII in the presence of a solvent to form a salt having Formula VIII 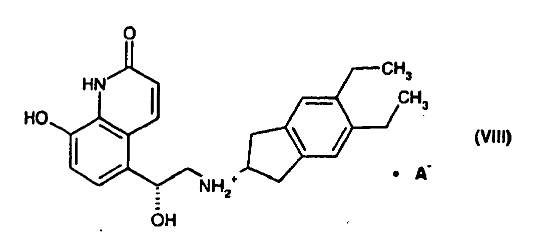 wherein A- is an anion; and
(j) treating the salt having formula VIII with an acid in the presence of a solvent to form a 5-[(R)-2-(5,6-diethyl-indan-2-ylamino)-1-hydroxy-ethyl]-8-hydroxy-(*1H*)-quinolin-2-one salt having formula IX 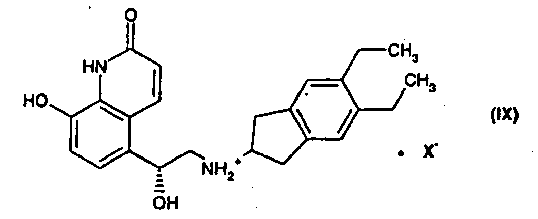 wherein X- is an anion.

## Patentansprüche

1. Verfahren zur Herstellung von 8-(substituiertes Oxy)-5-((R)-2-halogen-1-hydroxy-ethyl)-(1H)-chinolin-2-onen oder akzeptablen Solvaten hiervon durch Umsetzen eines 5-(α-Halogenacetyl)-8-(substituiertes Oxy)-(1H)-chinolin-2-ons mit einem Reduktionsmittel in Gegenwart eines chiralen Mittels und einer Base unter Bildung eines 8-(substituiertes Oxy)-5-((R)-2-halogen-1-hydroxyethyl)-(1H)-chinolin-2-ons, wobei das chirale Mittel eine Formel I oder II aufweist 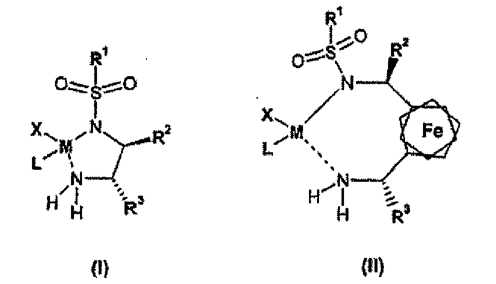 worin
M für Ru, Rh, Ir, Fe, Co oder Ni steht,
L für einen C₆-C₂₄-Arylrest oder einen C₆-C₂₄-Aryl-C₁-C₁₀-aliphatischen Rest steht, wobei in jedem Fall der Rest optional an ein Polymer gebunden ist;
X für Wasserstoff oder Halogen steht;
R¹ für einen C₁-C₁₀-allphatischen, C₃-C₁₀-cycloaliphatischen, C₃-C₁₀-cycloaliphatischen-C₁-C₁₀-aliphatischen, C₆-C₂₄-Aryl-, C₆-C₂₄-Aryl-C₁-C₁₀-aliphatischen Rest oder eine 4- bis 12-gliedrige heterocyclische Gruppe steht, der bzw. die in jedem Fall optional an ein Polymer gebunden ist; und R² und R³ für Phenyl stehen,
oder R² und R³ zusammen mit dem Kohlenstoffatom, an dem sie hängen, einen Cyclohexan- oder Cyclopentanring bilden.

2. Verfahren nach Anspruch 1, wobei das chirale Mittel die Formel I oder II aufweist, worin
M für Ruthenium steht,
L für Isopropylmethylbenzol, Benzol, Hexamethylbenzol oder Mesitylen steht;
X für Wasserstoff oder Halogen steht;
R¹ für Phenyl, 2- oder 3- oder 4-Pyridyl, 4'-Chlor-4-phenoxy-phenyl, 4-Phenoxy-phenyl, 5-Dimethylamino-1-naphthyl, 5-Nitro-1-naphthyl, 2-, 3-, 4-Nitrophenyl, 4-Vinylphenyl, 4-Biphenylyl, 9-Anthracenyl, 2-, 3- oder 4-Hydroxyphenyl, Tolyl, Phenanthryl, Benzo[1,3]-dioxol, Dimethyl-(naphthalin-1-yl)-amin, Mono- bis Tristrifluormethylphenyl, Chrysenyl, Perylenyl oder Pyranyl steht; und
R² und R³ beide für Phenyl stehen.

3. Verfahren nach Anspruch 1 oder 2, worin das chirale Mittel ein Mittel auf Rutheniumbasis ist und das Reduktionsmittel aus der Gruppe ausgewählt ist, die aus 2-Propanol, 3-Pentanol und Ameisensäure besteht.

4. Verfahren nach einem der vorhergehenden Ansprüche, worin das chirale Mittel RuCl[(1S,2S)-p-TsN-CH(C₆H₅)CH(C₆H₅)-NH₂](η⁶-p-Cymol) ist.

5. Verfahren nach einem der vorhergehenden Ansprüche, worin die verwendete Temperatur in einem Bereich von -10 bis 80 °C, vorzugsweise von 0 b is 50 °C liegt.

6. Verfahren nach einem der vorhergehenden Ansprüche, worin das 8-(substituiertes Oxy)-5-((R)-2-halogen-1-hydroxy-ethyl)-(1H)-chinolin-2-on 8-Phenylmethoxy-5-((R)-2-chlor-1-hydroxy-ethyl)-(1H)-chinolin-2-on ist.

7. Verfahren zur Herstellung von 5-[(R)-2-(5,6-Diethyl-indan-2-ylamino)-1-hydroxy-ethyl]-8-hydroxy-(1H)-chinolinon-2-on-salzen, das die folgenden Stufen umfasst:
(i) Umsetzen eines 5-(α-Halogenacetyl)-8-(substituiertes oxy)-(1H)-chinolin-2-ons mit einem Reduktionsmittel in Gegenwart eines chiralen Mittels und einer Base unter Bildung eines 8-(substituiertes Oxy)-5-((R)-2-halogen-1-hydroxy-ethyl)-(1H)-chinofin-2-ons, wobei das chirale Mittel eine Formel I oder II gemäß obiger Definition aufweist;
(ii) Behandeln des 8-(substituiertes Oxy)-5-((R)-2-halogen-1-hydoxy-ethyl)-(1H)-chinoln-2-ons mit einer Base in Gegenwart eines Lösemittels unter Bildung eines 8-(substituiertes Oxy)-5-(R)-oxiranyl-(1H)-chinolin-2-ons der Formel III 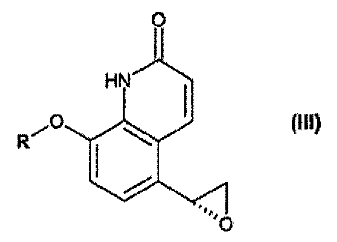 worin R für eine Schutzgruppe steht;
(iii) Umsetzen des 8-(substituiertes Oxy)-5-(R)-oxiranyl-(1H)-chinolin-2-ons der Formel III, worin R die oben angegebene Bedeutung besitzt, mit 2-Amino-(5-6-diethyl)-indan unter Bildung eines Verbindungen der Formeln IV, V und VI enthaltenden Reaktionsgemisches 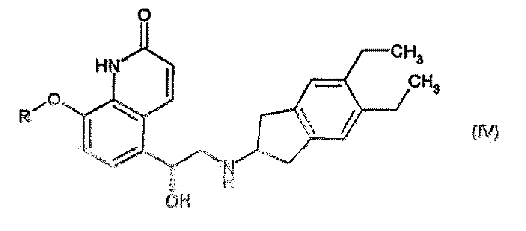 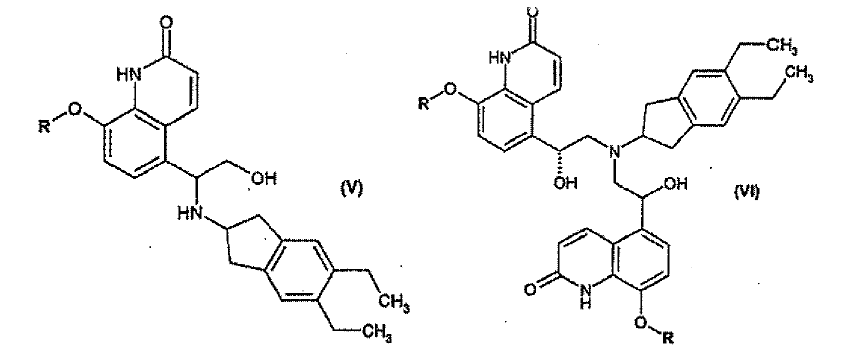 worin R für eine Schutzgruppe steht;
(iv) Behandeln des in Stufe (iii) hergestellten Reaktionsgemisches mit einer Säure in Gegenwart eines Lösemitteln unter Bildung eines entsprechenden Salzes;
(v) Isolieren und Kristallisieren eines Salzes der Formel VII 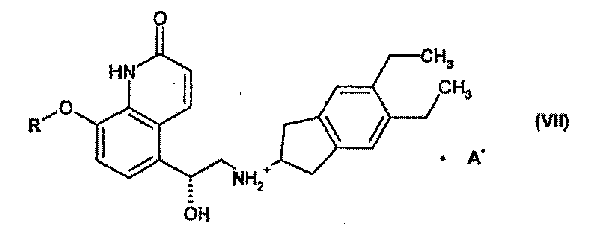 worin R für eine Schutzgruppe steht und A⁻ für ein Anion steht;
(vi) Entfernen der Schutzgruppe aus dem Salz der Formel VII in Gegenwart eines Lösemittels unter Bildung eines Salzes der Formel VIII 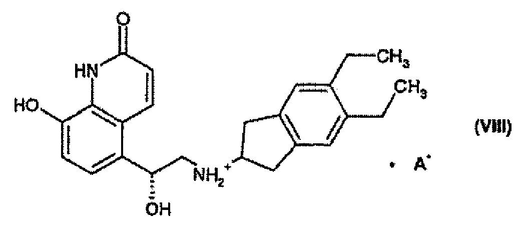 worin A⁻ für ein Anion steht; und
(vii) Behandeln des Salzes der Formel VIII mit einer Säure in Gegenwart eines Lösemittels unter Bildung eines 5-[(R)-(2-(5,6-diethyl-indan-2-ylamino)-1-hydroxy-ethyl]-8-hydroxy-(1H)-chinolin-2-on-salzes der Formel IX 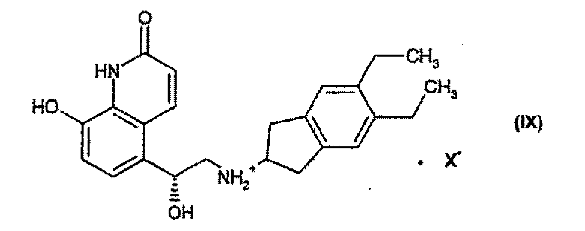 worin X für ein Anion steht.

8. Verfahren nach Anspruch 7, wobei das Reduktionsmittel Ameisensäure ist.

9. Verfahren nach Anspruch 7 oder 8, worin die in Stufe (ii) verwendete Base Ethoxid, Natriumhydroxid, Kaliumphosphat, Kaliumcarbonat, Kaliumhydrogencarbonat, Caesiumcarbonat oder ein Gemisch hiervon ist.

10. Verfahren zur Herstellung von 5-[(R)-2-(5,6-Diethyl-indan-2-ylamino)-1-hydroxy-ethyl]-8-hydroxy-(1H)-chinolin-2-on-salzen durch:
(a) Umsetzen
(i) eines 8-Hydroxy-(1H)-chinolin-2-ons mit einem Acylierungsmittel und einer Lewis-Säure unter Bildung eines 5-Acetyl-8-hydroxy-(1H)-chinolin-2-ons oder
(ii) eines 8-Hydroxy-(1H)-chinolin-2-ons mit einem Acylierungsmittel unter Bildung eines 8-Acetoxy-(1H)-chinolin-2-ons und in situ Behandeln des 8-Acetoxy-(1H)-chinolin-2-ons mit einer Lewis-Säure unter Bildung eines 5-Acetyl-8-hydroxy-(1H)-chinolin-2-ons, oder
(iii) eines 8-Acetoxy-(1H)-chinolin-2-ons mit einer Lewis-Säure unter Bildung eines 5-Acetyl-8-hydroxy-(1H)-chinolin-2-ons,
(b) Umsetzen des in Stufe (a) hergestellten 5-Acetyl-8-hydroxy-(1H)-chinolin-2-ons mit einer Verbindung der Formel R-Q in Gegenwart einer Base und eines Lösemittels unter Bildung eines 5-Acetyl-8-(substituiertes oxy)-(1H)-chinolin-2-ons, worin R für eine Schutzgruppe steht und Q für eine Abgangsgruppe steht;
(c) Umsetzen des 5-Acetyl-8-(substituiertes oxy)-(1H)-chinolin-2-ons mit einem Halogenierungsmittel in Gegenwart eines Lösemittels unter Bildung eines 5-(α-Halogenacetyl)-8-(substituiertes oxy)-(1H)-chinolin-2-ons;
(d) Umsetzen des 5-(α-Halogenacetyl)-8-(substituiertes oxy)-(1H)-chinolin-2-ons mit einem Reduktionsmittel in Gegenwart eines chiralen Mittels und einer Base unter Bildung eines 8-(substituiertes Oxy)-5-((R)-2-halogen-1-hydroxy-ethyl)-(1H)-chinolin-2-ons, wobei das chirale Mittel eine Formel I oder II gemäß obiger Definition aufweist;
(e) Behandeln des 8-(substituiertes Oxy)-5-((R)-2-ha)ogen-1-hydroxy-ethyl)-(1H)-chinolin-2-ons mit einer Base in Gegenwart eines Lösemittels unter Bildung eines 8-(substituiertes Oxy)-5-(R)-oxiranyl-(1H)-chinolin-2-one der Formal III 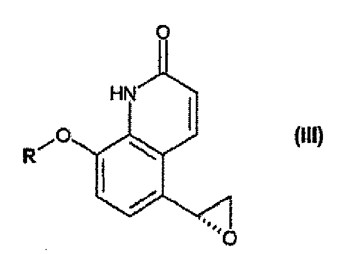 worin R für eine Schutzgruppe steht;
(f) Umsetzen des 8-(substituiertes Oxy)-5-(R)-oxiranyl-(1H)-chinolin-2-ons der Formel III, worin R die oben angegebene Bedeutung besitzt, mit einem 2-Amino-(5,6-diethyl)-indan unter Bildung eines Verbindungen der Formeln IV, V und VI enthaltenden Reaktionsgemisches 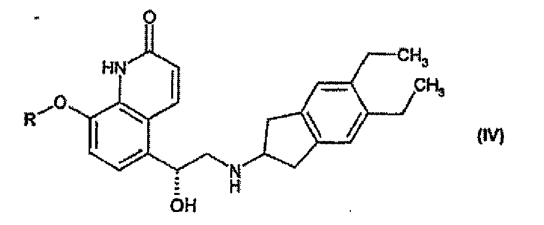 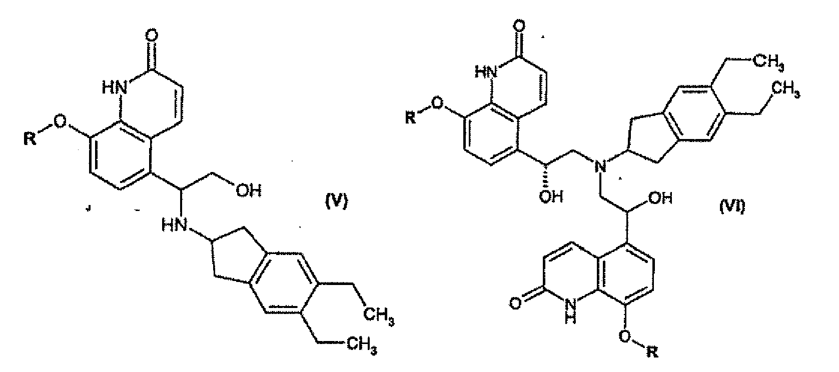 worin R für eine Schutzgruppe steht;
(g) Behandeln des in Stufe (f) hergestellten Reaktionsgemisches mit einer Säure in Gegenwart eines Lösemittels unter Bildung eines entsprechenden Salzes;
(h) isolieren und Kristallisieren eines Salzes der Formel VII 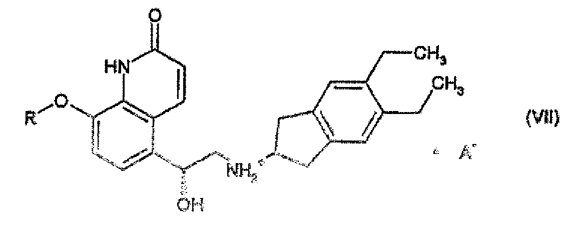 worin R für eine Schutzgruppe steht und A für ein Anion steht;
(i) Entfernen der Schutzgruppe aus dem Salz der Formel VII in Gegenwart eines Lösemittels unter Bildung eines Salzes der Formel VIII 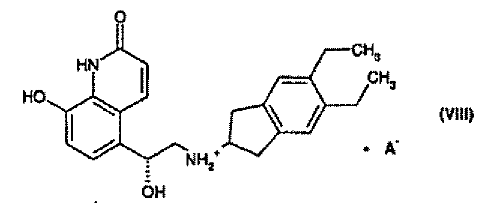 worin A⁻ für ein Anion steht und
(j) Behandeln des Salzes der Formel VIII mit einer Säure in Gegenwart eines Lösemittels zur Bildung eines 5-[(R)-2-(5,6-diethyl-indan-2-ylamino)-1-hydroxy-ethyl]-8-hydroxy-(1H)-chinolin-2-on-salzes der Formel IX 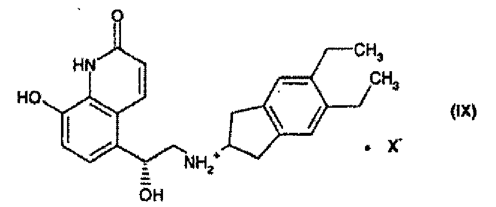 worin X⁻ für ein Anion steht.

## Revendications

1. Procédé de préparation de 8-(oxy substitué)-5-((R)-2-halogéna-1-hydroxy-éthyl)-1(*1-H*)-quinoléin-2-ones ou de produits de solvatation acceptables de ces dernières, comprenant la réaction d'une 5-(α-halogéno-acétyl)-8-(oxy substitué)-(*1H*)-quinoléin-2-one avec un agent réducteur en présence d'un agent chiral et d'une base pour former une 8-(oxy substitué)-5-((R)-2-halogéno-1-hydroxy-éthyl)-(*1H*)-quinoléin-2-one, ledit agent chiral ayant la formule I ou II 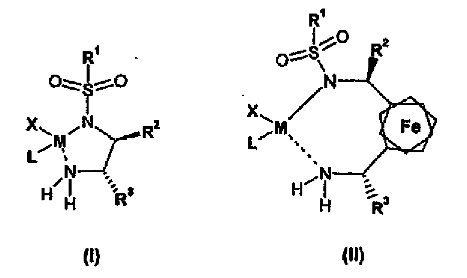 dans laquelle
M est Ru, Rh, Ir, Fe, Co ou Ni ;
L est un résidu aryle en C₆-C₂₄ ou (aryle en C₆-C₂₄)-(aliphatique en C₁-C₁₀), dans l'un et l'autre cas éventuellement lié à un polymère ;
X est un atome d'hydrogène ou un groupe halogéno ;
R¹ test un résidu aliphatique en C₁-C₁₀, cycloaliphatique en C₃-C₁₀, (cycloaliphatique en C₃-C₁₀)(aliphatique en C₁-C₁₀), aryle en C₆-C₂₄, (aryle en C₆-C₂₄)-(aliphatique en C₁-C₁₀) ou un groupe hétérocyclique à 4 à 12 chaînons, qui dans chaque cas est éventuellement lié à un polymère ; et
R² et R³ sont des groupes phényle,
ou bien R² et R³, ensemble avec l'atome de carbone auquel ils sont liés, forment un noyau cyclohexane ou cyclopentane.

2. Procédé selon la revendication 1, dans lequel l'agent chiral a la formule I ou II, dans laquelle :
M est le ruthénium ;
L est l'isopropylméthylbenzène, le benzène, l'hexaméthylbenzène ou le mesitylène ;
X est un atome d'hydrogène ou un groupe halogéno ;
R¹ est un radical phényle, 2-, 3- ou 4-pyridyle, 4'-chloro-4-phénoxy-phényle, 4-phénoxy-phényle, 5-diméthylamino-1-naphtyle, 5-nitro-1-naphtyle, 2-, 3-, 4-nitrophényle, 4-vinylphényle, 4-biphénylyle, 9-anthracényle, 2-, 3- ou 4-hydroxyphényle, tolyle, phénanthryle, benzo[1,3]dioxole, diméthyl-(naphtalèn-1-yl)-amine, (mono à tris)trifluorométhylphényle, chrysényle, pérylényle ou pyranyle ; et
R² et R³ sont tous les deux des groupes phényle.

3. Procédé selon la revendication 1 ou 2, dans lequel l'agent chiral est un agent à base de ruthénium, et l'agent réducteur est choisi dans le groupe consistant en le 2-propanol, le 3-pentanol et l'acide formique.

4. Procédé selon l'une quelconque des revendications précédentes, dans lequel l'agent chiral est RuC1[(1S,2S)-p-TsN-CH(C₆H₅)CH(C₆H₅)-NH₂](η⁶-p-cymène).

5. Procédé selon l'une quelconque des revendications précédentes, dans lequel la température utilisée est de -10 à 80°C, de préférence de 0 à 50°C.

6. Procédé selon l'une quelconque des revendications précédentes, dans lequel la -8-(oxy- substimé)-5-((R)-2-halogéno-1-hydroxy-éthyl)-(*1H*)-quinoléin-2-one est la 8-phénylméthoxy-5-((R)-2-chloro-1-hydroxy-éthyl)-(*1H*)-quinoléin-2-one.

7. Procédé de préparation de sels de 5-[(R)-2-(5,6-diéthyl-indan-2-ylamino)-1-hydroxy-éthyl]-8-hydroxy-(1H)-quinoléinone-2-one, comprenant :
(i) la réaction d'une 5-(α-halogénoacétyl)-8-(oxy substitué)-(*1H*)-quinoléin-2-one avec un agent réducteur en présence de l'agent chiral et d'une base pour former une 8-(oxy substitué)-5-((R)-2-halogéno-1-hydroxy-éthyl)-(*1H*)-quinoléin-2-one, ledit agent chiral ayant la formule I ou II telle que définie à la revendication 1 ;
(ii) le traitement de la 8-(oxy substitué)-5-((R)-2-halogéno-1-hydroxy-éthyl)-(*1H*)-quinoléin-2-one avec une base en présence d'un solvant pour former une 8-(oxy substitué)-5-(R)-oxiranyl-(1H)-quinolein-2-one de formule III 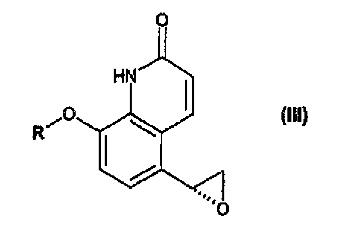 dans laquelle R est un groupe protecteur ;
(iii) la réaction de la 8-(oxy substitué)-5-(R)-oxiranyl-(1H)-quinoléin-2-one de formule III dans laquelle R est tel que défini ci-dessus, avec du 2-amino-(5,6-diéthyl)-indane pour former un mélange réactionnel contenant des composés ayant les formules IV, V et VI 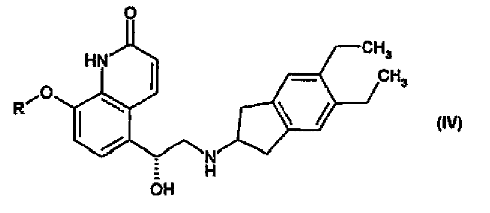 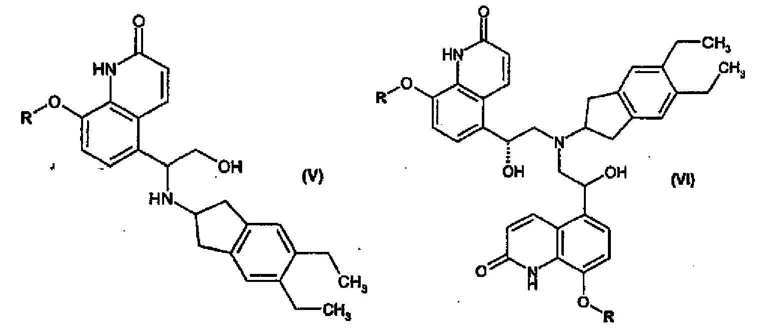 dans lesquelles R est un groupe protecteur ;
(iv) le traitement du mélange réactionnel préparé dans l'étape (iii) avec un acide en présence d'un solvant pour former un sel correspondant ;
(v) l'isolement et la cristallisation d'un sel de formule VII 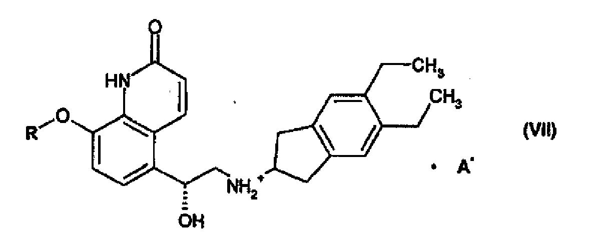 dans laquelle R est un groupe protecteur et A- est un anion ;
(vi) l'élimination du groupe protecteur du sel de formule VII en présence d'un solvant pour former un sel de formule VIII 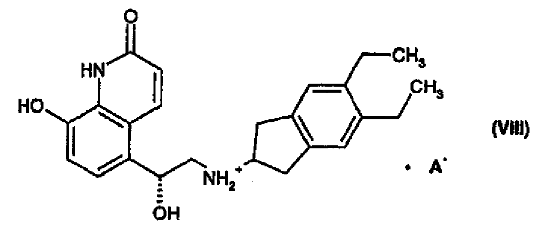 dans laquelle A⁻ est un anion ; et
(vii) le traitement du sel de formule VIII avec un acide en présence d'un solvant pour former un sel de 5-[(R)-2-(5,6-diéthyl-indan-2-ylamino)-1-hydroxy-éthyl]-8-hydroxy-(1H)-quinoléin-2-one de formule IX 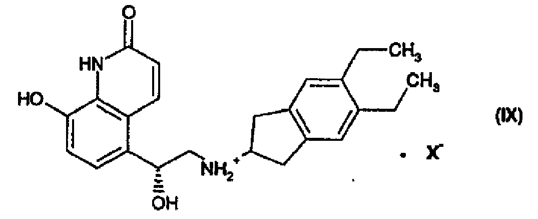 dans laquelle X⁻ est un anion.

8. Procédé selon la revendication 7, dans lequel l'agent réducteur est l'acide formique.

9. Procédé selon la revendication 7 ou 8, dans lequel la base utilisée dans l'étape (ii) est un éthoxyde, l'hydroxyde de sodium, le phosphate de potassium, le carbonate de potassium, l'hydrogénocarbonate de potassium, le carbonate de césium ou un mélange de ceux-ci.

10. Procédé de préparation de sels de 5-[(R)-2-(5,6-diéthyl-indan-2-ylamino)-1-hydroxy-éthyl]-8-hydroxy-(1H)-quinoléin-2-one, comprenant:
(a) la réaction
(i) de 8-hydroxy-(1H)-quinoléin-2-one avec un agent d'acylation et un acide de Lewis pour former la 5-acétyl-8-hydroxy-(1H)-quinoléin-2-one ; ou
(ii)de 8-hydroxy-(1H)-quinoléin-2-one avec un agent d'acylation pour former la 8-acétoxy-(1H)-quinoléin-2-one, et le traitement *in situ* de la 8-acétoxy-(1H)-quinoléin-2-one avec un acide de Lewis pour former la 5-acétyl-8-hydroxy-(1H)-quinoléin-2-one ; ou
(iii) de 8-acétoxy-(1H)-quinoléin-2-one avec un acide de Lewis pour former la 5-acétyl-8-hydroxy-(1H)-quinoléin-2-one ;
(b) la réaction de la 5-acétyl-8-hydroxy-(1H)-quinoléin-2-one préparée dans l'étape (a) avec un composé ayant la formule R-Q en présence d'une base et d'un solvant pour former une 5-acétyl-8-(oxy substitué)-(1H)-quinoléin-2-one où R est un groupe protecteur et Q est un groupe partant ;
(c) la réaction de la 5-acétyl-8-(oxy substitué)-(1H)-quinoléin-2-one en présence d'un agent d'halogénation en présence d'un solvant pour former une 5-(α-halogénoacétyl)-8-(oxy substitué)-(*1H*)-quinoléin-2-one ;
(d) la réaction de la 5-(α-halogénoacétyl)-8-(oxy substitué)-(*1H*)-quinoléin-2-one avec un agent réducteur en présence d'un agent chiral et d'une base pour former une 8-(oxy substitué)-5-((R)-2-halogéno-1-hydroxy-éthyl)-(*1H*)-quinoléin-2-one, ledit agent chiral ayant la formule I ou II telle que définie à la revendication 1 ;
(e) le traitement de la 8-(oxy substitué)-5-((R)-2-halogéno-1-hydroxy-éthyl)-(*1H*)-quinoléin-2-one avec une base en présence d'un solvant pour former une 8-(oxy substitué)-5-(R)-oxiranyl-(1H)-quinoléin-2-one de formule III 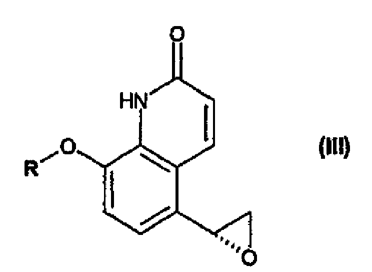 dans laquelle R est un groupe protecteur ;
(f) la réaction de la 8-(oxy substitué)-5-(R)-oxiranyl-(1H)-quinoléin-2-one de formule III dans laquelle R est tel que défini ci-dessus, avec du 2-amino-(5,6-diéthyl)-indane, pour former un mélange réactionnel contenant des composés ayant les formules IV, V et VI 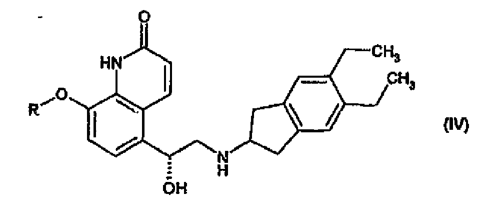 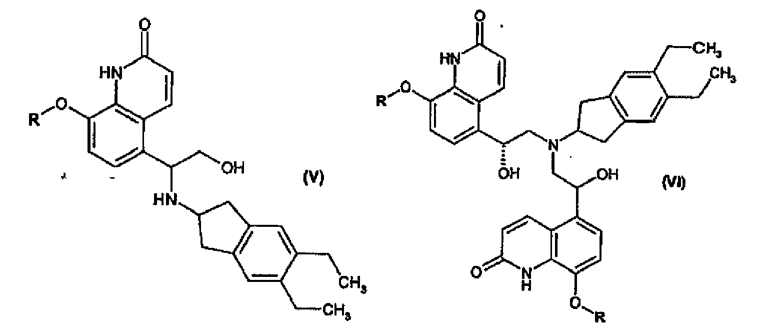 dans lesquelles R est un groupe protecteur ;
(g) le traitement du mélange réactionnel préparé dans l'étape (f) avec un acide en présence d'un solvant pour former un sel correspondant ;
(h) l'isolement et la cristallisation d'un sel de formule VII 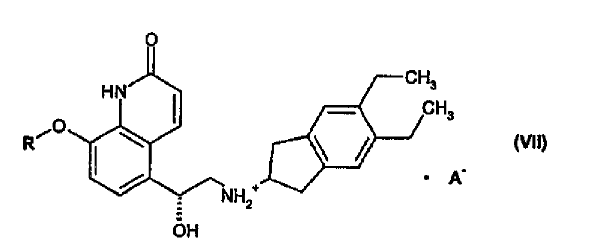 dans laquelle R est un groupe protecteur et A⁻ est un anion ;
(i) l'élimination du groupe protecteur du sel de formule VII en présence d'un solvant pour former un sel de formule VIII 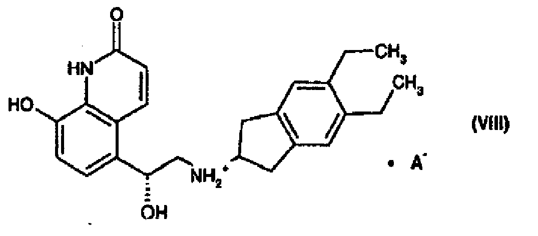 dans laquelle A⁻ est un anion ; et
(j) le traitement du sel de formule VIII avec un acide en présence d'un solvant pour former un sel de 5-[(R)-2-(5,6-diéthyl-indan-2-ylamino)-1-hydroxy-éthyl]-8-hydroxy-(1H)-quinaléin-2-one de formule IX 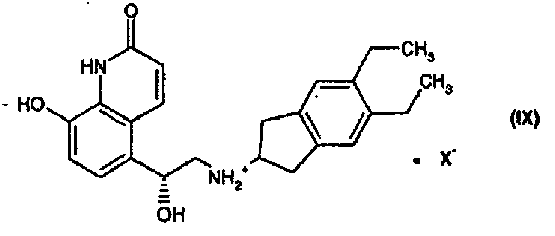 dans laquelle X- est un anion.
